# EUROPEAN PATENT APPLICATION

(11) **EP 4 230 228 A1**
(43) Date of publication of application: **23.08.2023**
(21) Application number: 21894289.4
(22) Date of filing: 02.09.2021
(51) Int. Cl.: A61L 2/10, A61L 2/24, A61L 9/20

(54) **INACTIVATION METHOD AND INACTIVATION SYSTEM**

(30) Priority: 20.11.2020 JP 2020193005
(71) Applicant: Ushio Denki Kabushiki Kaisha, Tokyo 100-8150 (JP)
(72) Inventor: OHASHI, Hiroyuki, Tokyo 100-8150 (JP); SUZUKI, Shinji, Tokyo 100-8150 (JP)
(74) Representative: Tomerius, Isabel
(86) International application number: PCT/JP2021/032280
(87) International publication number: WO 2022/107414

(57) **Abstract**

The present invention reduces a risk of infection with harmful microorganisms or viruses from person to person, and to effectively perform environmental sterilization. Provided is a method for inactivating microorganisms and/or viruses present in a specific space in which a person is present, by emitting light in a wavelength range for inactivating microorganisms and/or viruses in the space, the method comprising: a detection step of detecting a person present in an irradiation space irradiated with the light; an acquisition step of acquiring an accumulated irradiation amount which is an accumulated value of an irradiation amount of the light irradiated to the detected person in a period from a predetermined reference time point to a current time point; a determination step of determining an allowable maximum irradiation amount which is a maximum irradiation amount of the light allowed in the irradiation space by comparing an allowable limit value (TLV: Threshold Limit Value) of an irradiation amount of the light in the wavelength range with the accumulated irradiation amount; and an irradiation step of controlling a light source for emitting the light and irradiating the light into the irradiation space with the allowable maximum irradiation amount as an upper limit.

## Description

### TECHNICAL FIELD

The present invention relates to an inactivation method and an inactivation system for inactivating harmful microorganisms and/or viruses.

### BACKGROUND ART

A healthcare facility, schools, government offices, theaters, hotels, restaurants, or other facilities where people frequently gather, or enter and exit are in environments where microorganisms such as bacteria and mold are likely to propagate and where viruses are likely to spread. In particular, such a tendency becomes remarkable in a small space (a closed space such as a hospital room, a toilet, or an elevator) in the above-described facility and a space that is densely populated.

For example, harmful and highly infectious microorganisms or viruses, as a person infected with the viruses or the like enter or exit a predetermined space in a facility, will proliferate on a surface of a floor, a wall, or the like in the space or float in the space. Therefore, the viruses or the like will be infected in a next person who enters the space, and in some cases, an infectious disease may spread in the facility.

In order to improve the above-mentioned situations, in a facility where people (in some cases, animals) gather, or enter and exit, an action to disinfect harmful microorganisms as described above (for example, infectious microorganisms) and to inactivate viruses is required.

As for the surface surrounding the space, such as a floor or a wall, for example, a decontamination operation such as spraying alcohol or other disinfectant, wiping with a cloth or the like soaked in disinfectant, or irradiating with sterilizing ultraviolet light by an operator is performed. In addition, as for microorganisms, viruses, or the like suspended in the space, sterilization and inactivation by, for example, ultraviolet irradiation is performed.

Patent Literature Document 1 discloses, as a decontamination device for decontaminating a sealed chamber, a device for sterilizing a space to be decontaminated by irradiating the space with ultraviolet light (UVC light) when there is no user.

Ultraviolet light, depending on its wavelength, may adversely affect human or animal cells. For this reason, an allowable limit value is specified for an ultraviolet irradiation amount to a person, assuming a case where ultraviolet light are irradiated to the person.

Furthermore, in recent years, a system has been proposed which is for predicting an exposure amount for ultraviolet light contained in natural light and an exposure amount in the future.

Patent Literature Document 2 discloses an exposure amount estimation system that associates position information with environmental information (ultraviolet amount) at each position to predict previous and future exposure amount to ultraviolet radiation to an individual person from behavior information, such as movement of an individual person, the position information, and the environmental information. This system allows an individual person to develop measures to prevent and control ultraviolet exposure. In other words, even with ultraviolet light contained in natural light, individuals are also becoming more conscious of wanting to minimize their exposure.

### LISTING OF REFERENCES

### PATENT LITERATURE DOCUMENTS

Patent Literature Document 1: JP-201 7-528258A
Patent Literature Document 2: JP-5524741B

### SUMMARY OF THE INVENTION

### PROBLEMS TO BE SOLVED BY THE INVENTION

In the technique described in Patent Literature Document 1, after a person exits the space to be decontaminated, a surface surrounding the space and an environmental surface in the space are decontaminated.

However, in order to prevent a virus from being transmitted from a person infected with a harmful virus to another person, it is necessary to perform decontamination work more intensively on an area where the infected person has moved in the space where the infected person has entered or the environmental surface where the infected person has contacted. However, whether or not a person who has entered the space is infected with a harmful virus is rarely known at the time of entry into the space, and the infection is usually found later.

In addition, places where the infected person comes into contact or splashes from the infected person may be unexpected (unanticipated) places.

Therefore, it is difficult to track areas where the infected person has moved, environment surfaces where the infected person has contacted, or the like, to appropriately perform the decontamination work.

Further, as described above, the allowable limit value is specified for an ultraviolet irradiation amount to a person, and there is also a trend of increasing individual awareness of an exposure amount of ultraviolet light contained in natural light. In the case of sterilization and inactivation by ultraviolet irradiation, even if ultraviolet light emitted from an ultraviolet irradiation device is irradiated to a person, the intensity of ultraviolet light emitted from the ultraviolet irradiation device is often set to a low and constant value to ensure safety.

Accordingly, in the case of sterilization and inactivation of microorganisms and viruses by ultraviolet irradiation, the efficiency is not very high.

Therefore, an object of the present invention is to provide an inactivation method and an inactivation system capable of effectively performing environmental sterilization by reducing a risk of infection of harmful microorganisms or viruses from person to person.

### SOLUTION TO THE PROBLEMS

In order to solve the above problem, one aspect of an inactivation method according to the present invention is an inactivation method for inactivating microorganisms and/or viruses present in a specific space in which a person is present by emitting light in a wavelength range which inactivates microorganisms and/or viruses into the space, the method including: a detection step of detecting a person present in an irradiation space irradiated with the light; an acquisition step of acquiring an accumulated irradiation amount which is an accumulated value of an irradiation amount of the light irradiated to the detected person in a period from a predetermined reference time point to a current time point; a determination step of determining an allowable maximum irradiation amount which is a maximum irradiation amount of the light allowed in the irradiation space by comparing an allowable limit value (TLV: Threshold Limit Value) of an irradiation amount of the light in the wavelength range with the accumulated irradiation amount; and an irradiation step of controlling a light source for emitting the light and irradiating the light into the irradiation space with the allowable maximum irradiation amount as an upper limit.

Accordingly, an irradiation amount in an irradiation space can be controlled in accordance with an accumulated irradiation amount up to a current time point exposed to a person present in the irradiation space. Since an allowable maximum irradiation amount is determined by comparing an allowable limit value (TLV) with the accumulated irradiation amount, and a light is irradiated with the allowable maximum irradiation amount as an upper limit, it is possible to perform safe and effective inactivation for a person present in the irradiation space. For example, irradiating light until a determined allowable maximum irradiation amount is reached allows for environmental sterilization with maximum effectiveness.

In addition, in the above-described inactivation method, the light may be ultraviolet light in a wavelength range of 200 to 240 nm.

In this case, sterilization and inactivation can be effectively performed using light that has little adverse effect on human or animal cells.

Further, in the above-described inactivation method, in a case where a plurality of persons are detected in the detection step, in the acquisition step, the accumulated irradiation amount of each of the plurality of persons present in the irradiation space may be acquired, and in the determination step, an amount obtained by subtracting the largest accumulated irradiation amount from the accumulated irradiation amounts of the plurality of persons from the allowable limit value may be determined as the allowable maximum irradiation amount.

In this case, in a case where a plurality of persons having different behavior patterns are present in the same irradiation space, an inactivation effect can be maximized and light irradiation can be safely performed for each person.

Further, in the above-described inactivation method, the acquisition of the accumulated irradiation amount in the acquisition step and the determination of the allowable maximum irradiation amount in the determination step may be performed at a timing at which entry and exit of a person into and from the irradiation space are detected.

In this case, for example, even when a person in the irradiation space is replaced, it is possible to appropriately control an irradiation amount according to a person present in the irradiation space.

Further, in the above-described inactivation method, in the irradiation step, an illuminance of the light may be controlled or an irradiation time of the light may be controlled so that an irradiation amount of the light does not exceed the allowable maximum irradiation amount.

In this case, it is possible to appropriately control the illuminance of the light so that the irradiation amount of the light does not exceed the allowable maximum irradiation amount.

In addition, an aspect of the inactivation system according to the present invention is an inactivation system for inactivating microorganisms and/or viruses present in a specific space in which a person is present by emitting light into the space, comprising at least one light irradiation device, the device including: a light irradiation unit comprising a light source emitting light in a wavelength range which inactivates microorganisms and/or viruses; and
a control unit configured to control an irradiation of the light by the light source. The control unit includes: a detection unit configured to detect a person present in the irradiation space irradiated with the light from the light source; an acquisition unit configured to acquire an accumulated irradiation amount which is an accumulated value of an irradiation amount of the light irradiated to a person detected by the detection unit in a period from a predetermined reference time point to a current time point; and a determination unit configured to determine an allowable maximum irradiation amount which is a maximum irradiation amount of the light allowed in the irradiation space by comparing an allowable limit value (TLV: Threshold Limit Value) of an irradiation amount of the light in the wavelength range with the accumulated irradiation amount acquired by the acquisition unit, and wherein the control unit controls the light source to irradiate the light into the irradiation space with the allowable maximum irradiation amount determined by the determination unit as an upper limit.

Accordingly, an irradiation amount in an irradiation space can be controlled in accordance with an accumulated irradiation amount up to a current time point exposed to a person present in the irradiation space. Since an allowable maximum irradiation amount is determined by comparing an allowable limit value (TLV) with the accumulated irradiation amount, and a light is irradiated with the allowable maximum irradiation amount as an upper limit, it is possible to perform safe and effective inactivation for a person present in the irradiation space. For example, irradiating light until a determined allowable maximum irradiation amount is reached allows for environmental sterilization with maximum effectiveness.

In addition, in the above-described inactivation system, the light may be ultraviolet light in a wavelength range of 200 to 240 nm.

In this case, sterilization and inactivation can be effectively performed using light that has little adverse effect on human or animal cells.

Further, in the above-described inactivation system described above, in a case where a plurality of persons are detected by the detection unit, the acquisition unit may acquire the accumulated irradiation amount of each of the plurality of persons present in the irradiation space, and the determination unit may determine an amount obtained by subtracting the largest accumulated irradiation amount from the accumulated irradiation amounts of the plurality of persons acquired by the acquisition unit from the allowable limit value, as the allowable maximum irradiation amount.

In this case, in a case where a plurality of persons having different behavior patterns are present in the same irradiation space, an inactivation effect can be maximized and light irradiation can be safely performed for each person.

Further, in the above-described inactivation system, the acquisition unit may acquire the accumulated irradiation amount and the determination unit may determine the allowable maximum irradiation amount at a timing when the detection unit detects the entry and exit of a person into and from the irradiation space.

In this case, for example, even when a person in the irradiation space is replaced, it is possible to appropriately control an irradiation amount according to a person present in the irradiation space.

Further, in the above-described inactivation system, the control unit may control an illuminance of the light or may control an irradiation time of the light so that an irradiation amount of the light does not exceed the allowable maximum irradiation amount.

In this case, it is possible to appropriately control the illuminance of the light so that the irradiation amount of the light does not exceed the allowable maximum irradiation amount.

In addition, the above-described inactivation system may further include a recording device which is carried by a person moving in the specific space and records the accumulated irradiation amount of the person in a period from the reference time point to a current time point, wherein the acquisition unit may acquire the accumulated irradiation amount from the recording device carried by a person detected by the detection unit.

In this case, it possible to acquire the accumulated irradiation amount corresponding to each person from the recording device carried by the person present in the irradiation space. Therefore, a means for separately identifying an individual person is unnecessary.

Further, in the above-described inactivation system, the recording device may include a light sensor carried by a person moving in the specific space and configured to measure an illuminance of the light irradiated to the person, and the recording device may be a sensor device which calculates an irradiation amount of the light based on the illuminance of the light measured by the light sensor and an irradiation time of the light, and records the accumulated irradiation amount.

In this case, the recording device carried by each person can appropriately calculate and record the accumulated irradiation amount of each person.

Further, the above-described inactivation system may further include a recording device configured to record the accumulated irradiation amounts of all persons moving in the specific space in a time period from the reference time point to the current time point in association with personal identification information for identifying an individual person, wherein the detection unit may identify a person present in the irradiation space to acquire the personal identification information, and the acquisition unit may acquire, from the recording device, the accumulated irradiation amount associated with the personal identification information of a person identified by the detection unit.

In this case, it is possible to appropriately acquire the accumulated irradiation amount of a person present in the irradiation space.

Further, the above-described inactivation system may further include a light sensor carried by a person moving in the specific space and configured to measure an illuminance of the light irradiated to the person, the control unit may include a transmission unit configured to acquire an illuminance of the light from the light sensor carried by a person identified by the detection unit, calculate an irradiation amount of the light based on the acquired illuminance of the light and an irradiation time of the light, and transmit the irradiation amount to the recording device in association with the personal identification information of a person identified by the detection unit, and the recording device may record the irradiation amount of the light transmitted by the transmission unit by accumulating the accumulated irradiation amount recorded in association with the personal identification information.

In this case, the light irradiation device can appropriately calculate the irradiation amount to be exposed to each person present in the irradiation space of the light emitted from the light irradiation device and transmit the irradiation amount to the recording device. Therefore, the recording device can appropriately calculate and record the accumulated irradiation amount of each person.

Further, the above-described inactivation system may further include a distance acquisition unit configured to acquire a distance from the light source to an irradiation target of the light, and a light intensity acquisition unit configured to acquire a light intensity of the light emitted from the light source, wherein the control unit may include a transmission unit configured to calculate an illuminance of the light irradiated to a person identified by the detection unit based on a distance from the light source to a person identified by the detection unit and a light intensity acquired by the light intensity acquisition unit, calculate an irradiation amount of the light based on the calculated luminance of the light and an irradiation time of the light, and transmit the irradiation amount to the recording device in association with the personal identification information of a person identified by the detection unit, and the recording device may record an irradiation amount of the light transmitted by the transmission unit by accumulating the accumulated irradiation amount recorded in association with the personal identification information.

In this case, since the light irradiation device calculates an illuminance of light to be irradiated to each person present in the irradiation space of the light emitted from the light irradiation device, even when each person does not carry a sensor or the like for detecting the illuminance of light, the light irradiation device can appropriately calculate the amount of irradiation to be exposed to each person present in the irradiation space and transmit it to the recording device. Therefore, the recording device can appropriately calculate and record the accumulated irradiation amount of each person.

In addition, in the above-described inactivation system, the distance acquisition unit may acquire, as a distance from the light source to the irradiation target, a value obtained by subtracting a reference height, which is a height from a floor to the irradiation target, from a height from the floor to the light source.

In this case, a sensor for measuring the distance from the light source to each person is not required, and a simple system configuration can be realized.

Further, the above-described inactivation system may further include a plurality of the light irradiation devices in the specific space, wherein the accumulated irradiation amount recorded in the recording device is used as shared data of the control unit included in each of the plurality of light irradiation devices.

In this case, even in a case where each person moving in the specific space is exposed to light from a plurality of different light irradiation devices in a plurality of different spaces, each light irradiation device can accurately acquire the accumulated irradiation amount of each person.

In addition, in the above-described inactivation system, the recording device resets the recorded accumulated irradiation amount every predetermined period from the reference time point.

In this case, the light irradiation device can easily and appropriately compare the accumulated irradiation amount recorded in the recording device with the allowable limit value (TLV).

Furthermore, the above-described inactivation system may further include a display unit configured to display, for a person moving in the specific space, a time until the accumulated irradiation amount of the person reaches the allowable limit value.

In this case, an individual person can easily grasp a light irradiation state of the individual person.

In addition, in the above-described inactivation system, the light irradiation unit may include a housing having a light emitting window for emitting at least a part of the light emitted from the light source, and the light emitting window may be equipped with an optical filter which blocks transmission of UV-C wave on a longer side of a wavelength than 237 nm.

In this case, it is possible to irradiate only light in a wavelength range with little adverse effect on the human body or the animal.

Further, in the above-described inactivation system, the light source may be one of an excimer lamp and an LED

An excimer lamp and an LED are less susceptible to vibrations, atmospheric pressure changes, and thermal changes than low-pressure mercury lamps, which have been used as ultraviolet light sources in conventional inactivation device. That is, the illuminance of the emitted light is less likely to become unstable even when the light is subjected to vibration, atmospheric pressure change, or temperature change. Therefore, by using an excimer lamp or an LED as a light source, even when the light emitting device is used in an environment subjected to vibration, atmospheric pressure change, or temperature change, light can be stably emitted, and sterilization and inactivation can be appropriately performed.

In addition, in the above-described inactivation system, the light source may emit ultraviolet light with a center wavelength of 222 nm.

In this case, it is possible to effectively inactivate microorganisms and viruses while appropriately suppressing adverse effects on a human body caused by ultraviolet irradiation.

Furthermore, for example, in the above-described inactivation system, the light source may be an LED, and the LED may be one of an aluminum gallium nitride (AlGaN)-based LED, an aluminum nitride (AIN)-based LED, and a magnesium zinc oxide (MgZnO)-based LED.

In this case, ultraviolet light in a wavelength range of 200 to 240 nm, which have less adverse effects on a human body using an LED that is less susceptible to vibrations, atmospheric pressure changes, and thermal changes, can be emitted to appropriately inactivate microorganisms and viruses.

In addition, in the above-described inactivation system, the light source may be an LED, and the light irradiation unit may include a cooling member for cooling the LED.

In this case, a thermal rise of an LED can be appropriately suppressed, and a stable light can be emitted from an LED.

Further, in the above-described inactivation system, the light source may be an excimer lamp, and the light irradiation unit may include a housing which contains the excimer lamp and is made of a conductive metal.

In this case, it is possible to suppress a high-frequency noise generated from the excimer lamp from being transmitted to the outside of the housing. Thus, it is possible to suppress a control command to a control system provided outside the housing from being affected by the high-frequency noise from the excimer lamp, it is possible to suppress a failure of the control command.

### ADVANTAGEOUS EFFECTS OF THE INVENTION

According to the present invention, it is possible to reduce a risk of infection of harmful microorganisms or viruses from person to person, and to effectively perform environmental sterilization.

### BRIEF DESCRIPTION OF THE DRAWINGS

FIG. 1 is a diagram showing an ultraviolet absorption spectrum of a protein.
FIG. 2A is an example of a behavior pattern of a person in a facility.
FIG. 2B is an example of a behavior pattern of a person in a facility.
FIG. 3A is an example of a behavior pattern of a person between facilities.
FIG. 3B is an example of a behavior pattern of a person between facilities.
FIG. 4A is a diagram for explaining a relationship between an accumulated dose amount and an allowable ultraviolet irradiation amount.
FIG. 4B is a diagram for explaining a relationship between an accumulated dose amount and an allowable ultraviolet irradiation amount.
FIG. 5 is a configuration example of an inactivation system according to a first embodiment.
FIG. 6 is a flowchart showing an operation of an inactivation system according to a first embodiment.
FIG. 7 is a configuration example of an inactivation system according to modifications of a first embodiment.
FIG. 8 is a flowchart showing an operation of an inactivation system according to modifications of a first embodiment.
FIG. 9 is a flowchart showing an irradiation amount recording processing procedure of modifications of a first embodiment.
FIG. 10 is a configuration example of an inactivation system according to a second embodiment.
FIG. 11 is a flowchart showing an irradiation amount recording processing procedure according to a second embodiment.
FIG. 12 is a schematic diagram showing a configuration example of an ultraviolet irradiation unit.
FIG. 13 is a schematic diagram showing a configuration example of an excimer lamp.
FIG. 14 is a schematic diagram showing another example of an excimer lamp.
FIG. 15 is a schematic diagram showing another example of an excimer lamp.
FIG. 16 is a schematic diagram showing another example of an ultraviolet irradiation unit.

### DETAILED DESCRIPTION OF THE EMBODIMENTS

Hereinafter, embodiments of the present invention will be described with reference to the drawings.

In the present embodiment, an inactivation system will be described in which ultraviolet irradiation is performed in a specific space in which a person is present, and microorganisms and viruses present in the space are inactivated.

Note that "a specific space in which a person is present" is not limited to a space in which a person is actually present, and includes a space in which a person may enter and exit, but a person is not present. In addition, "inactivation" in the present embodiment refers to killing microorganisms or viruses (or loss infectivity or toxicity).

The specific space includes, for example, a space in a facility such as an office, a commercial facility, a medical facility, a station facility, a school, an office, a theater, a hotel, a restaurant, or the like, or a space in a vehicle such as an automobile, a train, a bus, a tax, an airplane, a ship, or the like. Note that the specific space may be a closed space such as a hospital room, a conference room, a toilet, or an elevator, or may be an unclosed space.

In addition, the specific space may be a space in which a person may move, and may be a single space provided in the above-described facility, vehicle, or the like, or may include a plurality of spaces.

The inactivation system according to the present embodiment is for, by irradiating ultraviolet light in a wavelength of 200 to 240 nm that has a small adverse effect on human and animal cells to an area in which a person in present, inactivating harmful microorganisms and viruses that exist on an object surface or a space within the area. The object includes a human body, an animal, and a thing.

In practice, a wavelength range of ultraviolet light used for decontamination (sterilization) applications is from 200 to 320 nm, and in particular, it is common to use ultraviolet light around 260 nm, which is highly absorbed by nucleic acids (DNA and RNA) possessed by microorganisms and viruses. However, such ultraviolet light of a wavelength range around 260 nm has a negative effect on humans and animals. For example, it may induce cancers due to erythema or skin DNA damages, or eye disorders (e.g., eye pain, hyperemia, corneal irritation).

Therefore, in a conventional ultraviolet irradiation system in which decontamination (sterilization) is performed using ultraviolet light in a wavelength range around 260 nm as described above, ultraviolet light is irradiated when there is no person or animal in view of the safety of persons or animals, and the emission of ultraviolet light is stopped when there is a person in the irradiation area.

However, a growth of harmful microorganisms in the space, a floatation of microorganisms and viruses, and an adhesion of microorganisms and viruses to a surface surrounding the space are often caused by an entry and exit of a person (infected person) or an animal having harmful microorganisms and viruses in the space. Therefore, essentially, in an ultraviolet irradiation system for decontamination (sterilization) application, it is efficient to decontaminate not only the space or surfaces surrounding the space, but also human or animal surfaces present in the area.

The present inventors, as a result of intensive studies, have found that light in a wavelength range of 200 to 240 nm is safe for humans and animals, and can kill microorganisms and inactivate viruses.

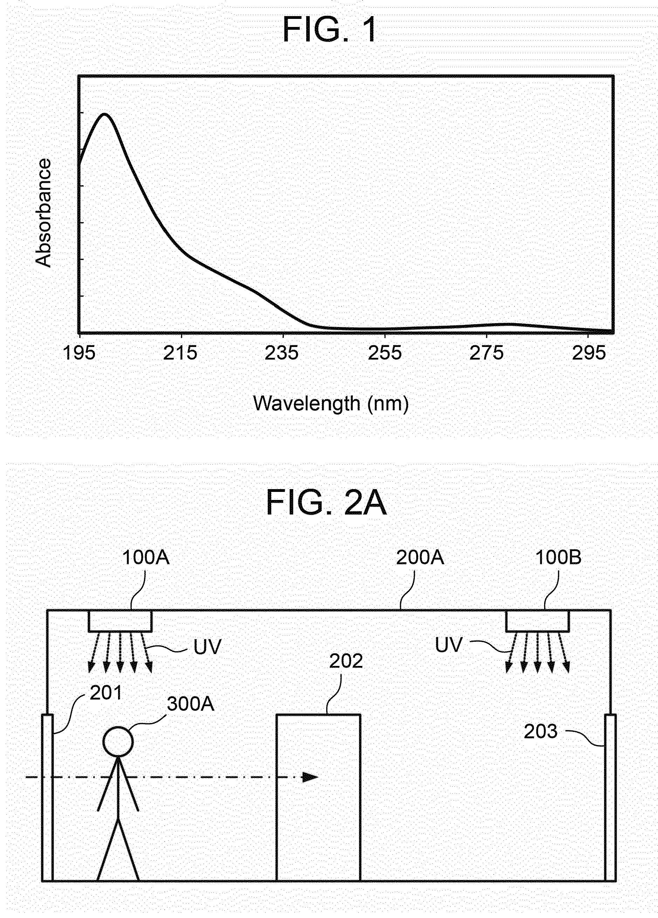

FIG. 1 is a diagram showing an ultraviolet absorption spectrum of a protein.

As shown in FIG. 1, it can be seen that the protein has an absorption peak at a wavelength of 200 nm, and ultraviolet light is hardly absorbed above a wavelength of 240 nm. That is, ultraviolet light with a wavelength of 240 nm or higher easily penetrate through a human skin and penetrate into the skin. Therefore, cells inside a human skin are easily damaged. In contrast, ultraviolet light with a wavelength of around 200 nm are absorbed by a skin surface of a human (e.g., the stratum corneum) and does not penetrate into a skin. It is therefore safe for a skin.

On the other hand, ultraviolet light with a wavelength less than 200 nm may generate ozone (O₃). This is because when ultraviolet light with a wavelength of less than 200 nm are irradiated into an atmosphere containing oxygen, the oxygen molecules are photolyzed to generate oxygen atoms, and ozone is generated by the bonding between the oxygen molecules and the oxygen atoms.

Therefore, the wavelength range of a wavelength of 200 to 240 nm can be said to be a wavelength range that is safe for humans and animals. Note that the wavelength range that is safe for humans and animals is preferably a wavelength of 200 to 237 nm, more preferably a wavelength of 200 to 235 nm, and even more preferably a wavelength of 200 to 230 nm.

The inactivation system according to the present embodiment is not, as in the prior art, configured so that ultraviolet light from the ultraviolet irradiation device does not irradiate a person, or so that an intensity of ultraviolet light emitted from the ultraviolet irradiation device is constant at a low value to ensure safety in the event that ultraviolet light are irradiated to a person, but is for actively irradiating ultraviolet light of a wavelength range safe for humans and animals as described above to a virus attached to a person or an object, a virus in a droplet emitted from a person, or an environmental surface where a virus from a person may attach.

Therefore, decontamination work can be more intensively performed on the infected person itself, on wearables (clothes, bags, or the like) worn by the infected person, on exhaled breath released by the infected person, on the area where the infected person has moved in the space where the infected person enters, or the environmental surface where the infected person has contacted, and thus an environmental sterilization effect can be improved. As a result, it is possible to prevent the virus from being transmitted from the person infected with the harmful virus to another person.

Accordingto ACGIH (American Conference of Governmental Industrial Hygienists) and JIS Z 8812 (Measuring Methods for Hazardous Ultraviolet Radiation), an allowable limit value (TLV: Threshold Limit Value) is defined for a daily (8 hours) ultraviolet irradiation amount to a human body.

Therefore, it is preferable to set the illuminance and the irradiation time of ultraviolet light so that the daily ultraviolet irradiation amount (integrated light amount) becomes equal to or less than the allowable limit value even in ultraviolet light in the wavelength range that is safe for humans as described above.

An example will be discussed in which one or more ultraviolet irradiation devices are provided in a specific space in which a person may enter and exit, and a person moving in the specific space is exposed to ultraviolet light emitted from the one or more ultraviolet irradiation devices.

In a space, a plurality of persons rarely behave according to the same behavior pattern, and a movement path is usually different for each person.

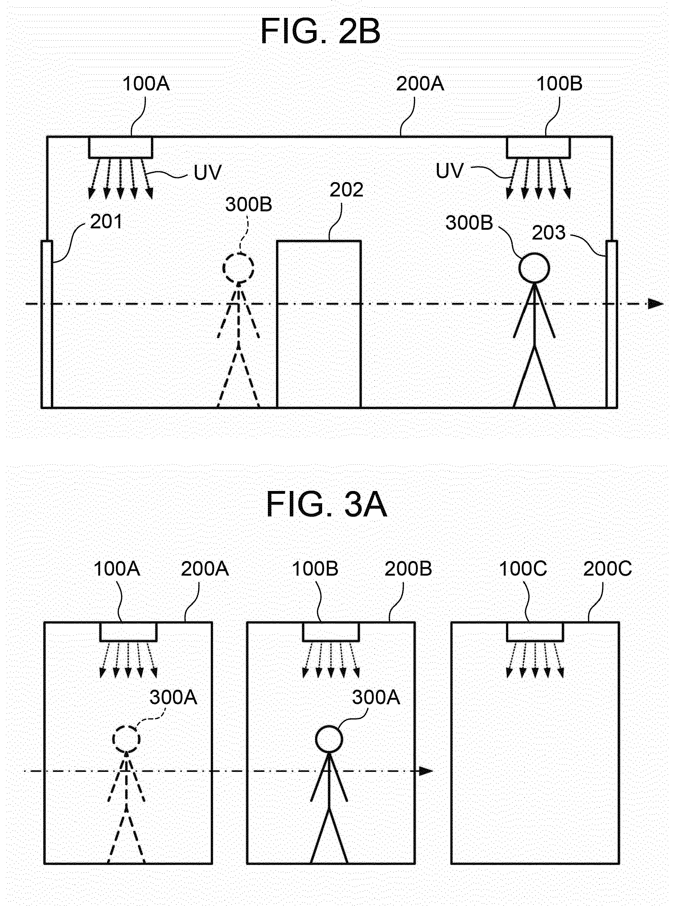

For example, as shown in FIGS.2A and 2B, it is assumed that two ultraviolet irradiation devices 100A and 100B are provided in one space (facility) 200A. Behavior patterns in the facility 200A are mostly different for each person.

For example, there is a behavior pattern, like the person 300A shown in FIG. 2A, that enters the facility 200A from the door 201, passes under the ultraviolet irradiation device 100A, and exits the facility 200A from the door 202, or a behavior pattern, like the person 300B shown in FIG. 2B, that enters the facility 200A from the door 201, passes under the ultraviolet irradiation device 100A, passes through the front part of the door 202, passes under the ultraviolet irradiation device 100B, and exits the facility 200A from the door 203, or another behavior pattern.

In this case, the person 300A is only irradiated with ultraviolet light from the ultraviolet irradiation device, but the person 300B is irradiated not only with ultraviolet light from the ultraviolet irradiation device 100A but also with ultraviolet light from the ultraviolet irradiation device 100B. Therefore, the accumulated ultraviolet irradiation amount (integrated light amount (accumulated dose amount) at the time of exiting the facility 200A may be different between the person 300A and the person 300B.

As described above, since the behavior patterns in a predetermined space are mostly different for each person, the ultraviolet irradiation amount (dose amount) may be different for each person.

Further, even if there is only one ultraviolet irradiation device provided in the facility 200A, since the behavior patterns in the facility 200A are mostly different for each person as described above, the number of times of entering the irradiation space irradiated with the ultraviolet light from the ultraviolet irradiation device is also often different for each person. Therefore, the ultraviolet irradiation amount may be also different for each person.

Further, even in a case where the ultraviolet irradiation device is provided in each of a plurality of spaces, a plurality of persons often behave according to the same behavior pattern, and the number of spaces that each person enter and exit is usually different.

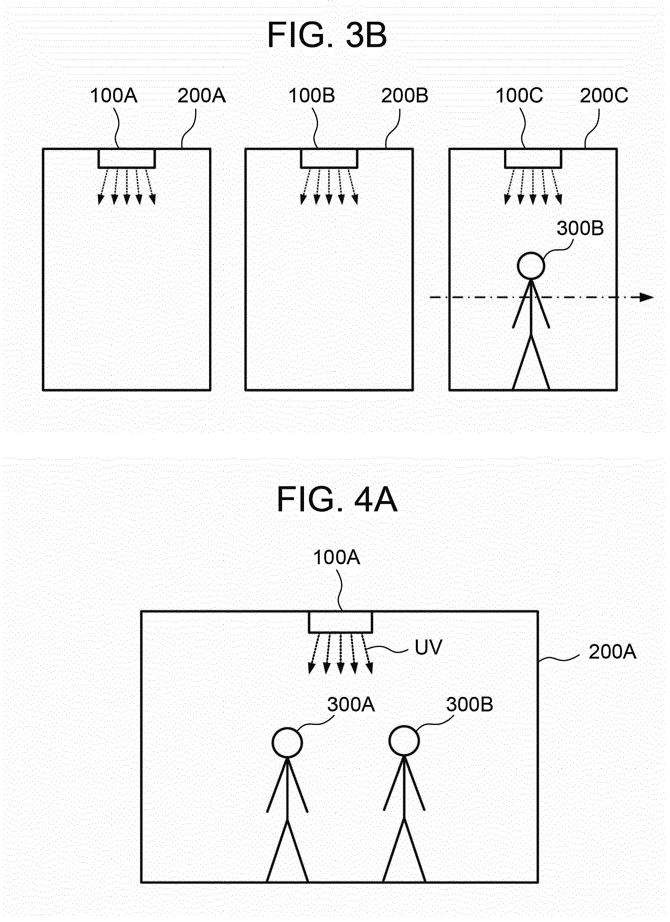

For example, as shown in FIGS. 3A and 3B, in a case where there are three spaces (facilities) 200A to 200C, and each ultraviolet irradiation device (100A to 100C) is provided one by one, there is a behavior pattern, like the person 300A shown in FIG. 3A, that enters and exits the facility 200A and the facility 200B, or a behavior pattern, like the person 300B shown in FIG. 3B, that enters and exits only the facility 200C, or another behavior pattern.

In this case, the person 300A is irradiated with ultraviolet light from the ultraviolet irradiation device 100A of the facility 200A and ultraviolet light from the ultraviolet irradiation device 100B of the facility 200B, but the person 300B is irradiated with only ultraviolet light from the ultraviolet irradiation device 100C of the facility 200C.

In this way, the person 300A and the person 300B have different opportunities for exposure to ultraviolet light.

Therefore, the ultraviolet irradiation amount of the person 300A and the person 300B may accidentally be the same amount, but is usually different.

In addition, even if both the persons 300A and 300B enter and exit the facility 200A and the facility 200B, differences in the behavior patterns of the persons 300A and 300B in each facility will result in differences in the ultraviolet irradiation amounts of the persons 300A and 300B.

As described above, in order to effectively perform environmental sterilization, it is desirable to appropriately irradiate ultraviolet light to a person, viruses attached to a person, viruses contained in a droplet released from a person, or an environmental surface to which viruses from a person may adhere.

Further, in order to safely irradiate ultraviolet light to a person, it is preferable to suppress an ultraviolet irradiation amount to be equal to or less than the allowable limit value (TLV) described above.

In other words, it is possible to achieve environmental sterilization that is safe for humans and maximize the effectiveness by irradiating the environmental surface until the ultraviolet irradiation amount reaches the allowable limit value (TLV).

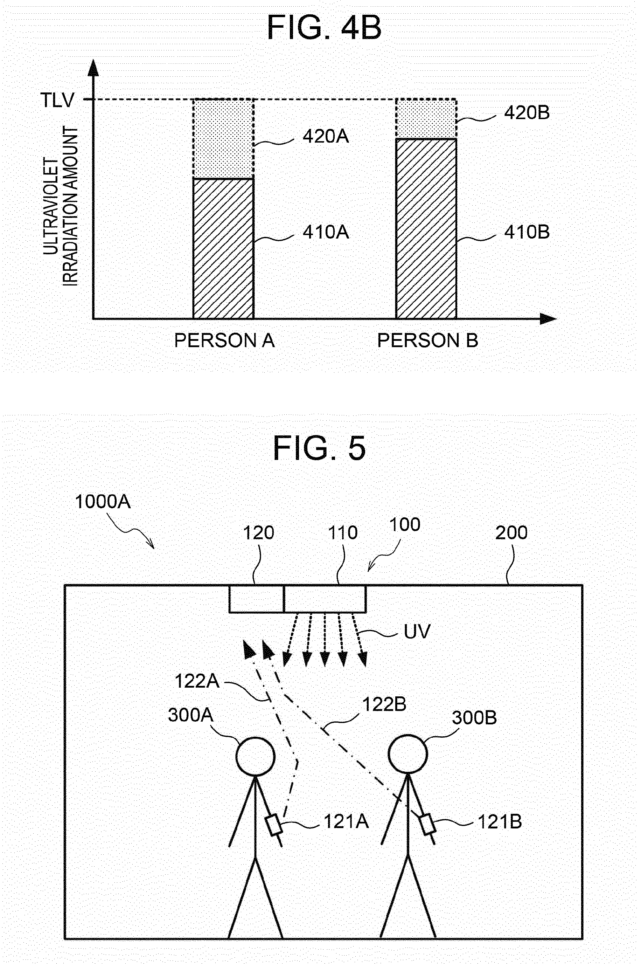

In a case where a plurality of persons whose accumulated ultraviolet irradiation amount (accumulated dose amount) is different from each other present in the irradiation space of ultraviolet light from the ultraviolet irradiation device, maximizing the inactivation effect and safely irradiating ultraviolet light to the persons is realized by irradiating ultraviolet light in the same irradiation space, in accordance with the person having the largest accumulated ultraviolet irradiation amount among the plurality of persons present in the same irradiation space, until the accumulated ultraviolet irradiation amount of the person reaches the allowable limit value (TLV). This point will be described with reference to FIGS. 4A and 4B.

As shown in FIG. 4A, it is assumed that the person 300A and the person 300B enter the facility 200A in which the ultraviolet irradiation device 100A is provided. In addition, it is assumed that the behavior patterns of the person 300A and the person 300B are different, and the accumulated ultraviolet irradiation amount (accumulated dose amount) of the person 300A and the person 300B before entering the facility 200A are different from each other.

Specifically, as shown in FIG. 4B, it is assumed that the accumulated ultraviolet irradiation amount 410A of the person 300A (person A) is smaller than the accumulated ultraviolet irradiation amount 410B of the person 300B (person B), and the remaining ultraviolet irradiation amount 420A until the accumulated ultraviolet irradiation amount 410A of the person 300A reaches the TLV is larger than the remaining ultraviolet irradiation amount 420B until the accumulated ultraviolet irradiation amount 410B of the person 300B reaches the TLV.

In this case, maximizing the effect of inactivation safely on persons in the facility 200A is realized by irradiating the person 300A and the person 300B with the remaining ultraviolet irradiation amount 420B of the person 300B in the facility 200A.

An inactivation method for safely maximizing an environmental sterilization effect on a person will be specifically described below.

### (First Embodiment)

FIG. 5 is a configuration example of an inactivation system 1000A according to the first embodiment. The inactivation system 1000A includes an ultraviolet irradiation device 100 provided in a facility 200 in a specific space, and an ultraviolet sensor 121A, 121B carried by the person 300A, 300B moving in the specific space.

The specific space is an area in which at least one space in which at least one ultraviolet irradiation device is provided exists. Although FIG. 5 illustrates only one facility 200 in the specific space, the specific space may include a space in which another ultraviolet irradiation device (not shown) is provided.

The ultraviolet irradiation device 100 includes an ultraviolet irradiator 110 including an ultraviolet light source that emits light in a wavelength range in which microorganisms and/or viruses are inactivated (in this case, ultraviolet light of a wavelength range of 200 to 240 nm), and a control unit 120 that controls irradiation of light by the ultraviolet irradiator 110.

The control unit 120 transmits a data request signal to the ultraviolet sensor 121A, 121B, and acquires ultraviolet data 122A, 122B transmitted by the ultraviolet sensor 121A, 121B in response to the data request signal. Then, the control unit 120 controls the irradiation of the light by the ultraviolet irradiator 110 based on the acquired ultraviolet data.

In the first embodiment, the ultraviolet sensor 121A, 121B is a sensor device (recording device) that calculates and records an ultraviolet irradiation amount emitted from the ultraviolet irradiation device 100 and irradiated to the person 300A, 300B.

The ultraviolet sensor 121A, 121B is, for example, wearable so as to be able to continuously sense ultraviolet light during a period in which the person 300A, 300B behaves in the specific space, and the person 300A, 300B behaving in the specific space wears the ultraviolet sensor 121A, 121B during a period in which the person 300A, 300B behaves in the specific space.

The ultraviolet sensor 121A, 121B measures the illuminance of ultraviolet light irradiated from the ultraviolet irradiation device 100, measures the irradiation time of the ultraviolet light, and calculates the ultraviolet irradiation amount (dose amount) based on the ultraviolet illuminance and the irradiation time. Then, the ultraviolet sensor 121A, 121B accumulates the ultraviolet irradiation amount for a specific period, and records the accumulated ultraviolet irradiation amount (accumulated dose amount) in the sensor as the ultraviolet data.

The specific period is a period from a predetermined reference time point (for example, a time point at which a person carrying the ultraviolet sensor enters the specific space) to a current time point. In other words, in the ultraviolet sensor 121A, 121B, while the person 300A, 300B is behaving in the specific space, the ultraviolet irradiation amount irradiated from at least one ultraviolet irradiation device provided in the specific space is accumulated and recorded.

Note that the ultraviolet data recorded in the sensor is reset every predetermined period (for example, one day) from the reference time point.

The control unit 120 acquires the accumulated ultraviolet irradiation amount of the person 300A, 300B from the ultraviolet sensor 121A, 121B carried by the person 300A, 300B present in the irradiation space of the ultraviolet irradiation device 100. Then, the control unit 120 compares the allowable limit value (TLV) of the ultraviolet irradiation amount with the acquired accumulated ultraviolet irradiation amount, and determines the maximum irradiation amount of ultraviolet light allowed (allowable maximum ultraviolet irradiation amount) in the facility 200. Specifically, the control unit 120 determines the amount obtained by subtracting the larger accumulated ultraviolet irradiation amount from the accumulated ultraviolet irradiation amount of the person 300A and the accumulated ultraviolet irradiation amount of the person 300B from the allowable limit value (TLV), as the allowable maximum ultraviolet irradiation amount. Then, the control unit 120 controls the ultraviolet light source of the ultraviolet irradiator 110 to irradiate the ultraviolet light into the facility 200 with the allowable maximum ultraviolet irradiation amount as an upper limit.

As described above, the inactivation system 1000A includes the recording device that is carried by a person who moves in the specific space and records the accumulated ultraviolet irradiation amount of the person from the reference time point to the current time point. The recording device is a sensor device (an ultraviolet sensor) that is carried by a person moving in a specific space, measures the illuminance of ultraviolet light irradiated to the person, calculates the ultraviolet irradiation amount based on the measured illuminance of the ultraviolet light and the irradiation time of the ultraviolet light, and accumulates and records the calculated amount.

The control unit 120 of the ultraviolet irradiation device 100 acquires the accumulated ultraviolet irradiation amount corresponding to each person from the recording device carried by the person detected in the irradiation space of the ultraviolet irradiation device 100, determines the allowable maximum ultraviolet irradiation amount to control the ultraviolet irradiation amount.

Next, an operation of the inactivation system 1000A according to the first embodiment will be described.

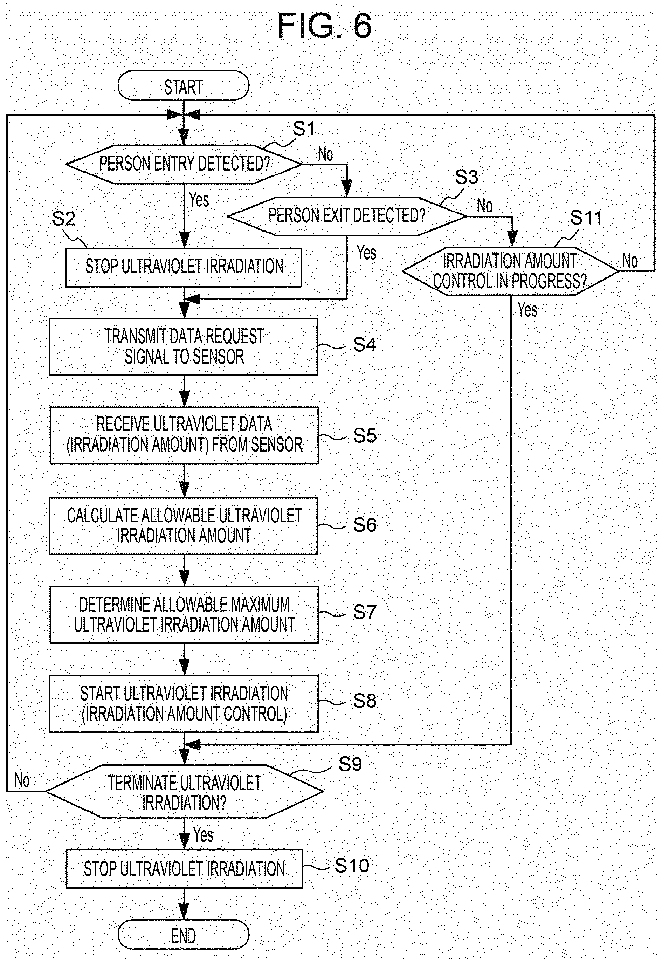

FIG. 6 is a flowchart of an ultraviolet irradiation processing procedure executed by the control unit 120. The processing illustrated in FIG. 6 is executed during normal ultraviolet irradiation by the ultraviolet irradiation device 100 (during normal lighting). The normal lighting refers to, for example, a method of irradiating ultraviolet light with an illuminance strong enough to sufficiently obtain an inactivating effect.

First, in step S1, the control unit 120 determines whether or not a person has entered a space (irradiation space) in which ultraviolet light are irradiated by the ultraviolet irradiation device 100. Specifically, the control unit 120 recognizes the presence of a person entering the irradiation space by a person recognition unit (not shown) included in the ultraviolet irradiation device 100. A human sensor or an image recognition unit may be used as the person recognition unit. When the image recognition unit is used, for example, an image captured by a camera, or the like is analyzed to detect a person present in the irradiation space.

The person recognition unit may be provided in the irradiation space independently of the ultraviolet irradiation device 100.

In a case where the control unit determines in step S1 that a new person has entered the irradiation space, it proceeds to step S2, stops the ultraviolet irradiation from the ultraviolet irradiator 110, and proceeds to step S4.

On the other hand, in a case where the control unit determines in step S1 that a new person has not entered the irradiation space, it proceeds to step S3, and determines whether or not there is a person exiting the irradiation space. Then, the control unit 120 proceeds to step S4 in a case where it determines that there is a person exiting the irradiation space, while proceeds to step S11 in a case where it determines that there is no person exiting the irradiation space.

In step S4, the control unit 120 transmits data request signals to the ultraviolet sensors carried by all persons present in the illumination space.

Next, in step S5, the control unit 120 receives ultraviolet data transmitted from the ultraviolet sensor that has received the data request signal. That is, the control unit 120 acquires the accumulated ultraviolet irradiation amount of all persons present in the irradiation space.

The accumulated ultraviolet irradiation amount acquired from the ultraviolet sensor is an ultraviolet irradiation amount accumulated by the time when the ultraviolet sensor receives the data request signal from the control unit 120, and includes, for example, an ultraviolet irradiation amount exposed in another space in a case where the person 300A, 300B present in the irradiation space was exposed to ultraviolet light in the another space before entering the facility 200.

In step S6, the control unit 120 calculates an ultraviolet irradiation amount allowed for each person present in the irradiation space (allowable ultraviolet irradiation amount). The ultraviolet irradiation device 100 previously stores the allowable limit value (TLV) corresponding to the wavelength of ultraviolet light (for example, a wavelength of 222 nm) emitted from the ultraviolet irradiator 110, and the control unit 120 calculates an amount obtained by subtracting the accumulated ultraviolet irradiation amount acquired in step S5 from the allowable limit value (TLV) as the allowable ultraviolet irradiation amount of each person.

Next, in step S7, the control unit 120 determines a maximum irradiation amount of the ultraviolet light allowed in the irradiation space (allowable maximum ultraviolet irradiation amount). Specifically, the control unit 120 selects the smallest allowable ultraviolet irradiation amount among the allowable ultraviolet irradiation amounts of the respective persons calculated in step S6, and determines the selected allowable ultraviolet irradiation amount as the allowable maximal ultraviolet irradiation amount. That is, an amount obtained by subtracting the largest accumulated ultraviolet irradiation amount among the accumulated ultraviolet irradiation amounts of the respective persons from the allowable limit value (TLV) is determined as the allowable maximum ultraviolet irradiation amount.

In step S8, the control unit 120 restarts the ultraviolet irradiation from the ultraviolet irradiator 110. At this time, the control unit 120 starts irradiation amount control for irradiating ultraviolet light for the irradiation space with the allowable maximum ultraviolet irradiation amount determined in step S7 as an upper limit. In the irradiation amount control, the control unit 120 may control the illuminance of the ultraviolet light or may control the irradiation time of the ultraviolet light so that the ultraviolet irradiation amount does not exceed the allowable maximum ultraviolet irradiation amount.

For example, in the irradiation amount control, ultraviolet light may be irradiated at a predetermined constant illuminance, and the ultraviolet irradiation may be terminated when the ultraviolet irradiation amount reaches the allowable maximum ultraviolet irradiation amount. In this case, the constant illuminance may be equal to the illuminance at the time of normal lighting, or may be lower than the illuminance at the time of normal lighting. In addition, in a case where the same irradiation space is irradiated by the plurality of ultraviolet light sources, the plurality of ultraviolet light sources may be individually turned on and off (for example, a predetermined number of ultraviolet light sources may be turned off).

Furthermore, the ultraviolet irradiation may be performed for a predetermined fixed irradiation time with an illuminance that does not exceed the allowable maximum ultraviolet irradiation amount.

In step S9, the control unit 120 determines whether to terminate the ultraviolet irradiation from the ultraviolet irradiator 110. Specifically, the control unit 120 determines whether or not the ultraviolet irradiation amount after the irradiation amount control is started in step S8 reaches the allowable maximal ultraviolet irradiation amount determined in step S7.

Then, in a case where the control unit 120 determines that the ultraviolet irradiation amount after the irradiation amount control is started in step S8 does not reach the allowable maximal ultraviolet irradiation amount determined in step S7, it determines to continue the ultraviolet irradiation and returns to step S1. On the other hand, in a case where the control unit 120 determines that the ultraviolet irradiation amount after the irradiation amount control is started in step S8 reaches the allowable maximal ultraviolet irradiation amount determined in step S7, it determines that the ultraviolet irradiation is terminated and proceeds to step S10.

In step S10, the control unit 120 ends the ultraviolet irradiation from the ultraviolet irradiator 110 and ends the process of FIG. 6.

In step S11, the control unit 120 determines whether or not the irradiation amount control started in step S8 is in progress. Then, the control unit 120 proceeds to step S9 in a case where it determines that the irradiation amount control is in progress, while returns to step S1 in a case where it determines that the irradiation amount control is not in progress.

For example, in a case where a person is not present in the facility 200 shown in FIG. 5 and ultraviolet irradiation (normal lighting) is performed at a predetermined intensity from the ultraviolet irradiator 110, the person 300A and the person 300B enter the facility 200 as shown in FIG. 5 (Yes in step S1 in FIG. 6), the control unit 120 temporarily stops the ultraviolet irradiation (step S2), and transmits a data request signal to the ultraviolet sensor 121A carried by the person 300A and the ultraviolet sensor 121B carried by the person 300B (step S4).

Then, the control unit 120 acquires the accumulated ultraviolet irradiation amount of the person 300A, 300B from the ultraviolet sensor 121A, 121B (step S5). It is assumed that the control unit 120 acquires the accumulated ultraviolet irradiation amount 410A, 410B shown in FIG. 4B, for example.

In this case, the control unit 120 calculates the ultraviolet irradiation amount 420A shown in FIG. 4B as the allowable ultraviolet irradiation amount of the person 300A, and calculates the ultraviolet irradiation amount 420B shown in FIG. 4B as the allowable ultraviolet irradiation amount of the person 300B (step S6). In addition, the control unit 120 determines the ultraviolet irradiation amount 420B, which is the smaller of the ultraviolet irradiation amount 420A and the ultraviolet irradiation amount 420B, as the allowable maximum ultraviolet irradiation amount in the facility 200 (step S7). Then, the control unit 120 restarts the ultraviolet irradiation so as not to exceed the ultraviolet irradiation amount 420B (step S8).

During the irradiation amount control, the entry and exit of a person into and from the irradiation space of ultraviolet light in the facility 200 are monitored, and the allowable maximum ultraviolet amount in the ultraviolet irradiation control is determined again at a timing when the entry and exit of a person is detected.

For example, in a case where the person 300B exits from the facility 200 (Yes in step S3), a data request signal is transmitted to the ultraviolet sensor 121A carried by the person 300A and the ultraviolet sensor 121B carried by the person 300B (step S4), and the latest accumulated ultraviolet irradiation amount of the person 300A is acquired again from the ultraviolet sensor 121A (step S5). The acquired accumulated ultraviolet irradiation amount is the accumulated ultraviolet irradiation amount before the person 200A enters the facility 200 plus the ultraviolet irradiation amount in the facility 200. The control unit 120 calculates the allowable ultraviolet irradiation amount of the person 300A based on the acquired latest accumulated ultraviolet irradiation amount (step S6), and determines again the calculated allowable ultraviolet irradiation amount of the person 300A as the allowable maximal ultraviolet irradiation amount in the facility 200 (step S7). Then, the control unit 120 performs ultraviolet irradiation so as not to exceed the newly determined allowable maximal ultraviolet irradiation amount (step S8).

In the above description, steps S1 and S3 in FIG. 6 correspond to a detection unit that detects a person present in the irradiation space, steps S4 and S5 correspond to an acquisition unit that acquires the accumulated ultraviolet irradiation amount (accumulated irradiation amount), and steps S6 and S7 correspond to a determination unit that determines the allowable maximum ultraviolet irradiation amount (allowable maximum irradiation amount).

As described above, in the inactivation system 1000A, the control unit 120 of the ultraviolet irradiation device 100 calls and acquires through communication the accumulated ultraviolet irradiation amount recorded in the recording device (ultraviolet sensor) carried by the person entering the irradiation space of the ultraviolet light emitted from the ultraviolet irradiator 110 of the ultraviolet irradiation device 100, and determines the remaining ultraviolet irradiation amount (allowable maximum ultraviolet irradiation amount) until reaching the allowable limit value (TLV). Then, the control unit 120 irradiates the irradiation space of the ultraviolet irradiator 110 with ultraviolet light within a range not exceeding the remaining ultraviolet irradiation amount.

In a case where a plurality of persons present in the irradiation space, the accumulated ultraviolet irradiation amount of each person is acquired from the ultraviolet sensor carried by each of the plurality of persons, and the amount obtained by subtracting the largest accumulated ultraviolet irradiation amount from the allowable limit value (TLV) is determined as the allowable maximum ultraviolet irradiation amount which is the maximum irradiation amount of ultraviolet light allowed in the irradiation space.

Therefore, even when the accumulated ultraviolet irradiation amount of each person is different, it is possible to perform safe and effective inactivation for each person.

In particular, it is possible to perform environmental sterilization which is safe for humans and has the greatest effect by irradiating ultraviolet light until the allowable maximum ultraviolet irradiation amount is reached.

### (Modifications of First Embodiment)

In the first embodiment, the case is described in which an ultraviolet sensor carried by an individual person calculates and records an accumulated ultraviolet irradiation amount (accumulated dose amount) of the individual person, and the ultraviolet irradiation device calls and obtains through communication the accumulated ultraviolet irradiation amount. However, the ultraviolet irradiation amount (dose amount) of an individual person may be recorded by an external server capable of communicating with the ultraviolet irradiation device. In this case, the ultraviolet irradiation device calculates the ultraviolet irradiation amount of each person, transmits the calculated ultraviolet irradiation amount to the server, and the server calculates and records the accumulated irradiation amount.

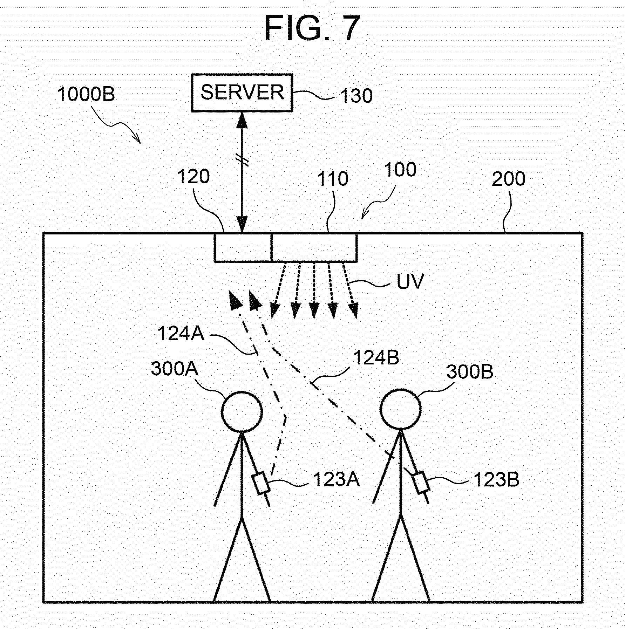

FIG. 7 is a configuration example of an inactivation system 1000B according to the modifications of the first embodiment.

The inactivation system 1000B includes the ultraviolet irradiation device 100 provided in the facility 200 in a specific space, an ultraviolet sensor 123A, 123B carried by a person 300A, 300B moving in the specific space, and a server 130 as a recording device.

The ultraviolet sensor 123A, 123B measures the illuminance of ultraviolet light emitted from the ultraviolet irradiation device 100 and irradiated to the person 300A, 300B.

Then, the ultraviolet sensor 123A, 123B transmits the measured ultraviolet illuminance as ultraviolet data 124A, 124B to the control unit 120 in response to the data request signal from the control unit 120 of the ultraviolet irradiation device 100.

The control unit 120 acquires the ultraviolet illuminance of the person 300A, 300B from the ultraviolet sensor 123A, 123B, and multiplies the acquired ultraviolet illuminance by the ultraviolet irradiation time to calculate the ultraviolet irradiation amount (dose amount) of the person 300A, 300B. Then, the control unit 120 transmits the calculated ultraviolet irradiation amount to the server 130 in association with personal identification information for identifying the person 300A, 300B.

The server 130 updates and records the accumulated ultraviolet irradiation amount by accumulating the ultraviolet irradiation amount (dose amount) of each person received from the control unit 120 to the accumulated ultraviolet irradiation amount (accumulated dose amount) recorded in association with the personal identification information.

In a case where the control unit 120 detects the person 300A, 300B present in the facility 200 as shown in FIG. 7, it identifies the individual of the person 300A, 300B, acquires the accumulated ultraviolet irradiation amount of the person 300A, 300B from the server 130, compares the allowable limit value of the ultraviolet irradiation (TLV) with the acquired accumulated ultraviolet irradiation amount, and determines the maximal irradiation amount of the ultraviolet light allowed in the facility 200 (allowable maximum ultraviolet irradiation amount). The method for determining the allowable maximum ultraviolet irradiation amount is the same as that of the first embodiment described above. Then, the control unit 120 controls the ultraviolet light source of the ultraviolet irradiator 110 to irradiate the inside of the facility 200 with the ultraviolet light with the allowable maximum ultraviolet irradiation amount as an upper limit.

In this way, the inactivation system 1000B includes a recording device for recording the accumulated ultraviolet irradiation amount from the reference point to the present point for all persons moving in the specific space. The recording device is a server 130 accessible by the control unit 120 of a plurality of the ultraviolet irradiation devices 100 provided in the specific space, and the accumulated ultraviolet irradiation amount recorded in the recording device is used as shared data of the control unit 120 of the plurality of ultraviolet irradiation devices 100.

The control unit 120 of the ultraviolet irradiation device 100 identifies a person present in the irradiation space of the ultraviolet irradiation device 100, acquires the accumulated ultraviolet irradiation amount of each identified person from the recording device, and determines the allowable maximum ultraviolet irradiation amount to perform irradiation amount control of ultraviolet light.

In addition, in the inactivation system 1000B, an individual person carries an ultraviolet sensor that measures the ultraviolet illuminance on the irradiation surface of the individual person of the ultraviolet light emitted from the ultraviolet irradiator 110, and the ultraviolet irradiation device 100 acquires the ultraviolet illuminance of the individual person from the ultraviolet sensor carried by the individual person. Then, the ultraviolet irradiation device 100 calculates the ultraviolet irradiation amount of each person based on the acquired ultraviolet illuminance and the ultraviolet irradiation time, and transmits the calculated amount to the recording device (server). As a result, the latest accumulated ultraviolet irradiation amount of each person is recorded in the recording device (server).

Next, an operation of the inactivation system 1000B will be described.

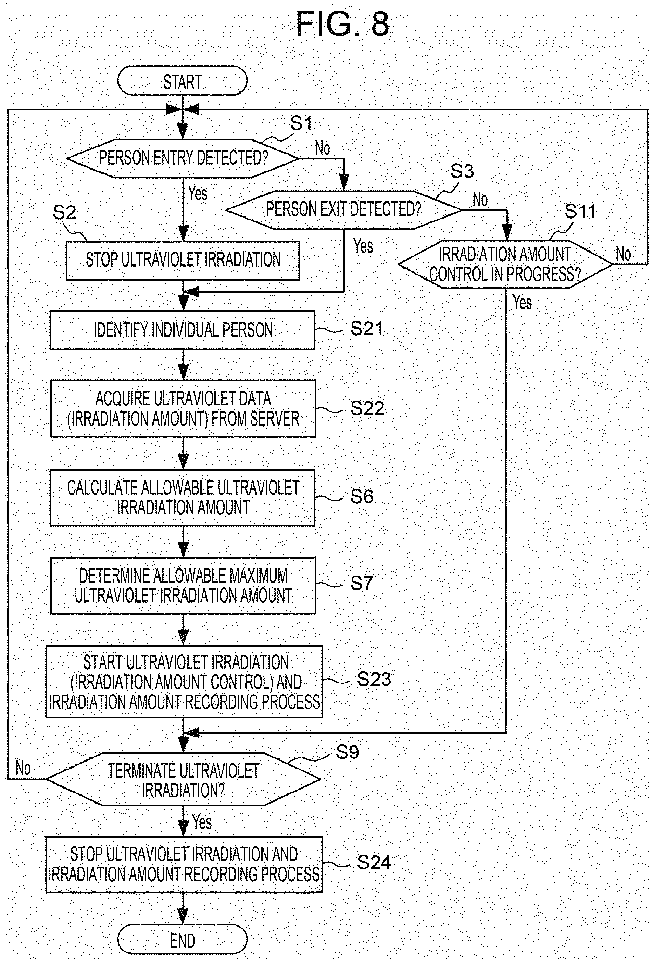

FIG. 8 is a flowchart of an ultraviolet irradiation processing procedure executed by the control unit 120. In FIG. 8, the same step numbers are assigned to the portions that execute the same processing as the ultraviolet irradiation processing shown in FIG. 6 described above, and the different portions of the processing will be mainly described below.

In step S21, the control unit 120 identifies an individual person of a person present in an illumination space and acquires individual identification information. Specifically, the control unit 120 identifies a person present in the irradiation space by unshown individual identification means included in the ultraviolet irradiation device 100. As the individual identification means, facial identification unit means be used, or ID information of an ultraviolet sensor or a smart phone or the like carried by each person may be used.

Note that the individual identification means may be provided in the ultraviolet irradiation device 100 or may be provided in the irradiation space independently of the ultraviolet irradiation device 100.

In step S22, the control unit 120 acquires the accumulated ultraviolet irradiation amount of the person identified in step S21 from the server 130, and proceeds to step S6.

In step S23, the control unit 120 starts irradiation amount control for irradiating ultraviolet light into the irradiation space with the allowable maximum ultraviolet irradiation amount determined in the step S7 as an upper limit. In addition, in step S23, the control unit 120 calculates the ultraviolet irradiation amount received by the person present in the irradiation space by the irradiation amount control, and starts an irradiation amount recording process for calculating and recording the accumulated ultraviolet irradiation amount in the server.

In step S24, the control unit 120 ends the irradiation amount control by the ultraviolet irradiator 110 and ends the above-described irradiation amount recording process.

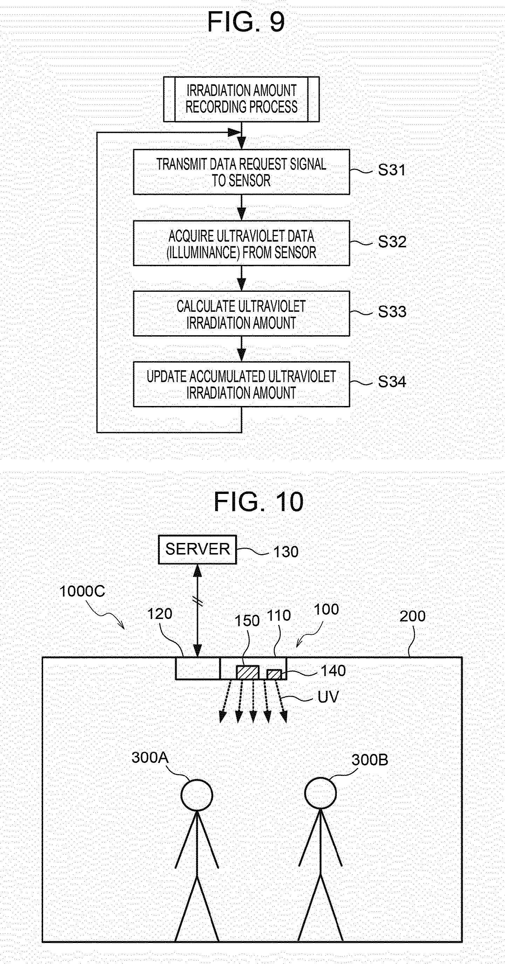

FIG. 9 is a flowchart illustrating an irradiation amount recording process started in step S23 of FIG. 8.

First, in step S31, the control unit 120 transmits a data request signal to the ultraviolet sensors carried by all persons present in the illumination space.

Next, in step S32, the control unit 120 receives the ultraviolet data transmitted from the ultraviolet sensor in response to the data request signal. That is, the control unit 120 acquires the ultraviolet illuminance of all persons present in the irradiation space.

Next, in step S33, the control unit 120 multiplies the ultraviolet illuminance acquired in step S32 by the ultraviolet irradiation time to calculate the ultraviolet irradiation amount (dose amount) of each person. The calculated ultraviolet irradiation amount (dose amount) is associated with the individual identification information by the individual identification means.

Next, in step S34, the control unit 120 transmits the ultraviolet irradiation amount calculated in step S33 to the server 130. Then, the transmitted ultraviolet irradiation amount of the individual person is accumulated in the accumulated ultraviolet irradiation amount of the corresponding individual person already recorded in the server 130, and the accumulated ultraviolet irradiation amount of the individual person is updated.

The irradiation amount recording process shown in FIG. 9 is repeatedly executed until the irradiation amount recording process is stopped in step S24 of FIG. 8.

For example, in a case where no person present in the facility 200 shown in FIG. 7 and the ultraviolet irradiation (normal lighting) is performed at a predetermined intensity from the ultraviolet irradiator 110, when the person 300A and the person 300B enter the facility 200 as shown in FIG. 7 (Yes in step S1 in FIG. 8), the control unit 120 temporarily stops the ultraviolet irradiation (step S2), identifies the individual of the person 300A, 300B (step S21), and acquires the accumulated ultraviolet irradiation amount of the person 300A, 300B from the server 130 (step S22). It is assumed that the control unit 120 acquires the accumulated ultraviolet irradiation amount 410A, 410B illustrated in FIG. 4B, for example.

In this case, the control unit 120 calculates the ultraviolet irradiation amount 420A shown in FIG. 4B as the allowable ultraviolet irradiation amount of the person 300A, and calculates the ultraviolet irradiation amount 420B shown in FIG. 4B as the allowable ultraviolet irradiation amount of the person 300B (step S6). In addition, the control unit 120 determines the ultraviolet irradiation amount 420B, which is the smaller of the ultraviolet irradiation amount 420A and the ultraviolet irradiation amount 420B, as the allowable maximum ultraviolet irradiation amount in the facility 200 (step S7). Then, the control unit 120 restarts the ultraviolet irradiation so as not to exceed the ultraviolet irradiation amount 420B (step S8).

During the irradiation amount control, the control unit 120 transmits a data request signal to the ultraviolet sensor 123A, 123B carried by the person 300A, 300B (step S31 in FIG. 9), and acquires the ultraviolet illuminance irradiated to the person 300A, 300B from the ultraviolet sensor 123A, 123B (step S32). Then, the control unit 120 calculates the ultraviolet irradiation amount based on the acquired ultraviolet illuminance and the irradiation time (step S33), and transmits the calculated ultraviolet irradiation amount to the server 130 in association with the personal identification information of the person 300A, 300B (step S34).

The server 130 updates and records the accumulated ultraviolet irradiation amount of the person 300A, 300B using the ultraviolet irradiation amount transmitted from the control unit 120.

In the above description, steps S1, S3 and S21 of FIG. 8 correspond to a detection unit that detects a person present in the irradiation space and specifies the person, step S22 corresponds to an acquisition unit that acquires the accumulated ultraviolet irradiation amount (accumulated irradiation amount), and steps S6 and S7 correspond to a determination unit that determines the allowable maximum ultraviolet irradiation amount (allowable maximum irradiation amount). Further, the processing of FIG. 9 corresponds to a transmission unit that calculates the ultraviolet irradiation amount of the person identified in the irradiation space and transmits the calculated ultraviolet irradiation amount to the recording device (server) in association with the personal identification information.

As described above, in the inactivation system 1000B, the control unit 120 of the ultraviolet irradiation device 100 calls and acquires through communication the accumulated ultraviolet irradiation amount of the individual person who has entered the irradiation space of the ultraviolet light emitted from the ultraviolet irradiator 110 of the ultraviolet irradiation device 100 from the server 130, and determines the remaining ultraviolet irradiation amount (allowable maximum ultraviolet irradiation amount) until reaching the allowable limit value (TLV). Then, the control unit 120 irradiates the irradiation space of the ultraviolet irradiator 110 with ultraviolet light within a range not exceeding the remaining ultraviolet irradiation amount.

As described above, the accumulated ultraviolet irradiation amount of each person may be recorded in the recording device (server) shared by the control unit 120 of the plurality of ultraviolet irradiation device 100, instead of being recorded in the recording device (ultraviolet sensor) carried by each person as in the first embodiment described above. Also in this case, as in the first embodiment described above, it is possible to appropriately determine the allowable maximum ultraviolet irradiation amount, and it is possible to perform safe for each person and effective inactivation.

### (Second Embodiment)

In the first embodiment and the modifications thereof described above, it is assumed that a person moving in a specific space carries an ultraviolet sensor. Therefore, in a case where a person does not wear the ultraviolet sensor (for example, when the ultraviolet sensor is forgotten at home or the like), the inactivation method cannot be applied.

In the second embodiment, an inactivation method will be described that can safely maximize the environmental sterilization effect for a person even if the person does not carry the ultraviolet sensor.

FIG. 10 is a configuration example of an inactivation system 1000C according to the second embodiment.

The inactivation system 1000C includes an ultraviolet irradiation device 100 provided in a facility 200 in a specific space, and a server 130 as a recording device.

Further, the ultraviolet irradiation device 100 includes a light intensity sensor (light intensity acquisition unit) 140 that measures the light intensity of the ultraviolet light emitted from the ultraviolet light source included in the ultraviolet irradiator 110, and a distance sensor (distance acquisition unit) 150 that measures the distance from the ultraviolet light source to the irradiation target (person).

In a case where an individual person carries an ultraviolet sensor as in the first embodiment, ultraviolet data from the ultraviolet sensor is information corresponding to each person. On the other hand, in a case where the ultraviolet sensor is not used, the ultraviolet irradiation device 100 needs to acquire the ultraviolet data corresponding to each person.

In the second embodiment, the control unit 120 calculates the ultraviolet illuminance of ultraviolet light emitted from the ultraviolet irradiator 110 on the irradiated surface of an individual person based on the light intensity of the ultraviolet light measured by the light intensity sensor 140 and the distance to the irradiation target measured by the distance sensor 150. Then, the control unit 120 multiplies the calculated ultraviolet illuminance by the ultraviolet irradiation time to calculate the ultraviolet irradiation amount (dose amount) of the individual person. Then, the control unit 120 transmits the calculated ultraviolet irradiation amount to the server 130 in association with the personal identification information for identifying the individual person.

The server 130 updates and records the accumulated ultraviolet irradiation amount by accumulating the ultraviolet irradiation amount (dose amount) of each person received from the control unit 120 to the accumulated ultraviolet irradiation amount (accumulated dose amount) recorded in association with the personal identification information.

In a case where the control unit 120 detects the person 300A, 300B present in the facility 200 as shown in FIG. 10, it identifies the individual of the person 300A, 300B, acquires the accumulated ultraviolet irradiation amount of the person 300A, 300B from the server 130, compares the allowable limit value (TLV) of the irradiation amount of ultraviolet light with the acquired accumulated ultraviolet irradiation amount, and determines the maximal irradiation amount of ultraviolet light allowed in the facility 200 (allowable maximum ultraviolet irradiation amount). The method for determining the allowable maximum ultraviolet irradiation amount is the same as that of the first embodiment described above. Then, the control unit 120 controls the ultraviolet light source of the ultraviolet irradiator 110 to irradiate into the facility 200 with the ultraviolet light with the allowable maximum ultraviolet irradiation amount as an upper limit.

As described above, the inactivation system 1000C includes the recording device that records the accumulated ultraviolet irradiation amount from the reference time point to the current time point of all persons moving in the specific space, similarly to the inactivation system 1000B described above. The recording device is a server 130 accessible by the control unit 120 of the plurality of ultraviolet irradiation devices 100 provided in the specific space, and the accumulated ultraviolet irradiation amount recorded in the recording device is used as shared data of the control unit 120 of the plurality of ultraviolet irradiation devices 100.

The control unit 120 of the ultraviolet irradiation device 100 identifies a person present in the irradiation space of the ultraviolet irradiation device 100, acquires the accumulated ultraviolet irradiation amount of each identified person from the recording device, and determines the allowable maximum ultraviolet irradiation amount to control the ultraviolet irradiation amount.

In the inactivation system 1000C, unlike the above described inactivation system 1000B, the ultraviolet irradiation device 100 includes the light intensity sensor 140 and the distance sensor 150, and calculates the ultraviolet illuminance of the ultraviolet light emitted from the ultraviolet irradiator 110 on the irradiation surface of the individual person based on the light intensity of the ultraviolet light measured by the light intensity sensor 140 and the distance to the irradiation target measured by the distance sensor 150. Then, the ultraviolet irradiation device 100 calculates the ultraviolet irradiation amount of the individual person based on the calculated ultraviolet illuminance and the ultraviolet irradiation time, and transmits the calculated amount to the recording device (server). As a result, the latest accumulated ultraviolet irradiation amount of each person is recorded in the recording apparatus (server).

Next, an operation of the inactivation system 1000C will be described.

The ultraviolet irradiation processing procedure executed by the control unit 120 of the inactivation system 1000C is the same as the processing procedure shown in FIG. 8. However, the irradiation amount recording process started in S23 of FIG. 8 is different from the modifications of the first embodiment.

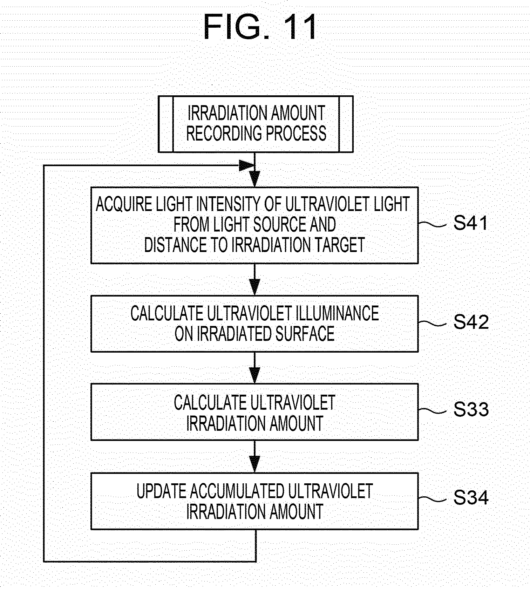

FIG. 11 is a flowchart showing an irradiation amount recording processing procedure. In FIG. 11, portions that perform the same processing as the irradiation amount recording processing shown in FIG. 9 described above are denoted by the same step numbers, and the different portions of the processing will be mainly described below.

In step S41, the control unit 120 acquires the light intensity of the ultraviolet light emitted from the ultraviolet irradiator 110 measured by the light intensity sensor 140, and acquires the distance from the ultraviolet irradiator 110 to the person present in the irradiation space measured by the distance sensor 150.

In step S42, the control unit 120 calculates the ultraviolet illuminance on the ultraviolet irradiation surface of each person present in the irradiation space based on the ultraviolet light intensity and the distance acquired in the step S41.

As described above, in the inactivation system 1000C, the ultraviolet irradiation device 100 includes the light intensity sensor 140 and the distance sensor 150, and calculates the ultraviolet illuminance of the ultraviolet light emitted from the ultraviolet irradiator 110 on the irradiation surface of the individual person.

As described above, the ultraviolet illuminance irradiated to the individual person may be calculated by the ultraviolet irradiation device 100 instead of being measured by the ultraviolet sensor carried by the individual person as in the modifications of the first embodiment described above. In this case as well, as in the first embodiment described above, since the accumulated ultraviolet irradiation amount of each person can be recorded in the server 130, the allowable maximum ultraviolet irradiation amount can be appropriately determined, and thus, it is possible to perform safe and effective inactivation for each person.

In the second embodiment, the distance from the ultraviolet light source to the irradiation target (person) is measured using the distance sensor 150, but in a case where the irradiation space is a relatively narrow space, the height of the person entering the irradiation space may be uniformly set to a predetermined reference height (for example, 180cm), and the value obtained by subtracting the reference height from the installation height of the ultraviolet irradiator 110 from the floor surface may be used for calculating the ultraviolet illuminance as the distance from the ultraviolet light source to the irradiation target (person).

In a case where the irradiation space is a large space, when a person is present at the end of the irradiation space, an angle formed between a line connecting the ultraviolet irradiator 110 and the person and a normal line from the ultraviolet irradiator 110 to the floor surface increases, and a distance from the ultraviolet light source to the person becomes larger than a distance from the ultraviolet light source to a person at the center of the irradiation space (directly below the ultraviolet light source). Therefore, if the distance from the ultraviolet light source to the irradiation target (person) is made uniform, a calculation error of the ultraviolet illuminance becomes large. Therefore, in a case where the irradiation space is a large space, it is preferable to calculate the ultraviolet illuminance using the distance measured by the distance sensor 150 as described above.

Further, in the modifications of the first embodiment and the second embodiment described above, the case is described in which the server 130 is equipped separately from the plurality of ultraviolet irradiation devices 100 provided in the specific space, but one of the plurality of ultraviolet irradiation devices 100 may have the function of the server 130 (recording device). In this case, the ultraviolet irradiation device 100 having the function of the server 130 may be configured to be able to communicate with other ultraviolet irradiation devices 100.

Further, in each of the above-described embodiments, the allowable ultraviolet irradiation amount of each person may be displayed for each person by the display unit. Alternatively, in a case where the intensity of ultraviolet light emitted from the ultraviolet irradiation device 100 in each facility 200 is constant, the allowable ultraviolet irradiation time may be displayed for each person based on the allowable ultraviolet irradiation amount of each person. The display unit may be provided in each facility 200 or may be carried by each person.

As a result, it is possible for an individual person to grasp his/her ultraviolet irradiation state in a predetermined period of time.

In each of the above described embodiments, in a case where the ultraviolet irradiation is performed by the blinking lighting that repeatedly turns on and off the light source continuously, "control of the irradiation time" also includes controlling the total ultraviolet irradiation time (total time during which the light source is turned on) by changing the number of times of repetition and/or the time ratio between the turn on and the turn off.

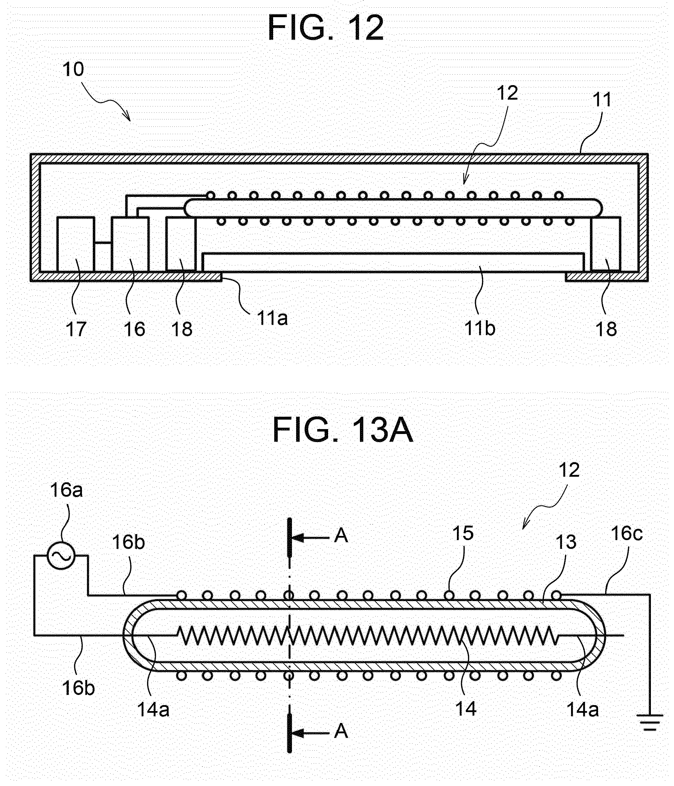

FIG. 12 is a schematic diagram showing a configuration example of the ultraviolet irradiation device 100 described above.

FIG. 12 mainly shows portions related to the ultraviolet irradiator 110 and the control unit 120. Hereinafter, the portions related to the ultraviolet irradiator 110 and the control unit 120 will be collectively referred to as an ultraviolet irradiation unit 10.

The ultraviolet irradiation unit 10 includes a housing 11 made of a conductive metal, and an ultraviolet light source 12 contained in the housing 11.

The ultraviolet light source 12 may be, for example, a KrCI excimer lamp that emits ultraviolet light with a center wavelength of 222 nm. Note that the ultraviolet light source 12 is not limited to a KrCI excimer lamp, and may be any light source that emits ultraviolet light in a wavelength range of 200 to 240 nm.

Further, the ultraviolet irradiation unit 10 includes a power supply unit 16 that supplies power to the excimer lamp 12, and a control unit 17 that controls irradiation and non-irradiation of the excimer lamp 12, the amount of ultraviolet light emitted from the excimer lamp 12, or the like. The control unit 17 corresponds to the above-described control unit 120 and has the above-described various functions.

The excimer lamp 12 is supported by a supporting part 18 in the housing 11.

The housing 11 is formed with an aperture 11a that serves as a light emitting window. The aperture 11a is provided with a window member 11b. The window member 11b may include, for example, an ultraviolet transmissive member made of quartz-glass, an optical filter that blocks unwanted light, or the like.

A plurality of excimer lamps 12 may be arranged in the housing 11. The number of excimer lamps 12 is not particularly limited.

As the optical filter, for example, a wavelength-selective filter that transmits light in a wavelength range 200 to 237 nm and cuts light having another UV-C wavelength range (238 to 280 nm light) may be used.

As the wavelength-selective filter, for example, a dielectric multilayer film filter including HfO₂ layers and SiO₂ layers may be used.

As the wavelength-selective filter, an optical filter having a dielectric multilayer film formed of SiO₂ layers and Al₂O₃ layers may also be used.

As described above, by providing the optical filter in the light emitting window, even when harmful light is emitted from the excimer lamp 12 to a person, it is possible to more reliably suppress the light from leaking out of the housing 11.

Hereinafter, a configuration example of the excimer lamp 12 used as an ultraviolet light source in the ultraviolet irradiation unit 10 will be specifically described.

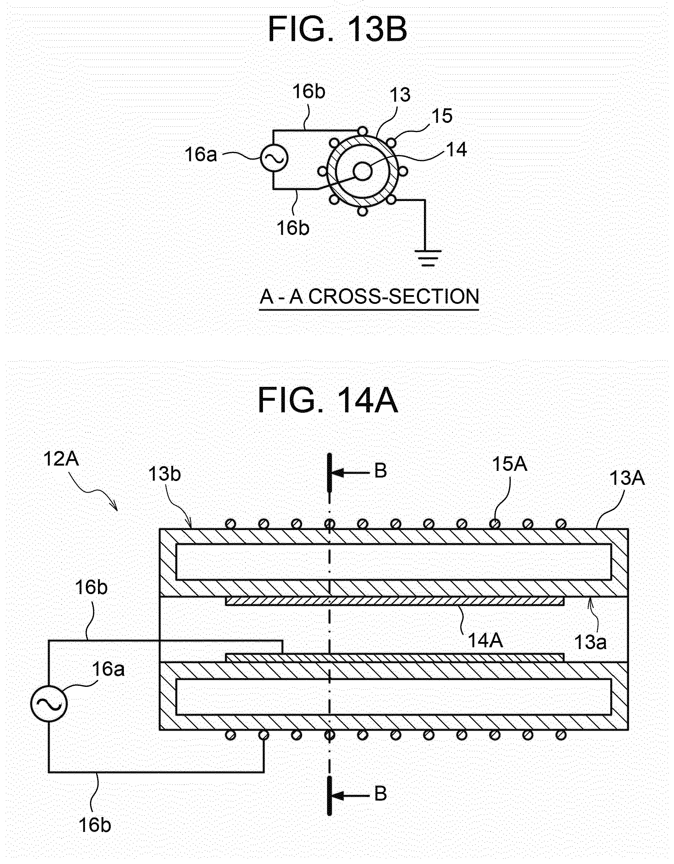

FIG. 13A is a schematic cross-section view of the excimer lamp 12 in the tube axial direction, and FIG. 13B is an A-A cross-section view of FIG. 13A.

As shown in FIGS. 13A and 13B, the excimer lamp 12 includes an elongated circular tube-shaped discharge vessel 13 both ends of which are hermetically sealed. The discharge vessel 13 is made of, for example, a light-transmitting dielectric material such as synthetic quartz glass or fused quartz glass that transmits ultraviolet light. A discharge space is formed in the discharge vessel 13, and a noble gas and a halogen gas are enclosed in the discharge space as a barrier discharge gas (hereinafter, also referred to as "discharge gas") for generating ultraviolet light. In the present embodiment, krypton (Kr) is used as the noble gas, and chlorine gas (Cl₂) is used as the halogen gas.

As the discharge gas, a mixed gas of krypton (Kr) and bromine (Br₂) may be used. In this case, the excimer lamp (KrBr excimer lamp) emits ultraviolet light with a center wavelength of 207 nm.

In addition, a first electrode (internal electrode) 14 is arranged in the discharge space inside the discharge vessel 13. The internal electrode 14 is a coiled electrode formed by winding a metal wire made of a metal having electrical conductivity and heat resistance, such as tungsten, into a coil shape by a coil diameter smaller than the inner diameter of the discharge vessel 13. The internal electrode 14 extends along the central axis (tube axis) of the discharge vessel 13 and is arranged so as not to come into contact with the inner peripheral surface of the discharge vessel 13.

Further, one end of an internal electrode lead member 14a is electrically connected to each of both ends of the internal electrode 14. The other end part of the internal electrode lead member 14a protrudes outward from the outer end surface of the discharge vessel 13.

A second electrode (external electrode) 15 is provided on the outer peripheral surface of the discharge vessel 13. The external electrode 15 is, for example, a reticulated electrode composed of metal stands made of a metal having electrical conductivity and heat resistance such as tungsten. The external electrode 15 is provided so as to extend along the outer peripheral surface of the discharge vessel 13 in the central axis direction of the discharge vessel 13. In the excimer lamp 12 shown in FIGS. 13A and 13B, the external electrode 15, which is a reticulated electrode, has a cylindrical outer shape and is provided in a state of being in close contact with the outer peripheral surface of the discharge vessel 13.

With such a configuration, in the discharge space, a discharge region is formed in a region where the internal electrode 14 and the external electrode 15 face each other via a tube wall (dielectric material wall) of the discharge vessel 13.

Further, one end of the external electrode 15 and the other end of the one inner electrode lead member 14a are each connected to a high frequency power supply 16a equipped with power supply unit 16 (see FIG. 12) via the power supply line 16b. The high frequency power supply 16a is a power supply capable of applying a high frequency voltage between the inner electrode 14 and the outer electrode 15.

One end of a lead wire 16c is electrically connected to the other end of the external electrode 15, and the other end of the lead wire 16c is grounded. That is, the external electrodes 15 are grounded via the lead wire 16c. In the excimer lamp 12 shown in FIGS. 13A and 13B, one of the inner electrode lead members 14a is integrated with the feed line 16b.

When high frequency power is applied between the internal electrode 14 and the external electrode 15, a dielectric barrier discharge is generated in the discharge space. By this dielectric barrier discharge, the atoms of the discharge gas (barrier discharge gas) enclosed in the discharge space are excited, and an excited dimer (exciplex) is generated. When the excited dimer returns to its original state (ground state), a unique emission (excimer emission) occurs. That is, the discharge gas is a gas for excimer light emission.

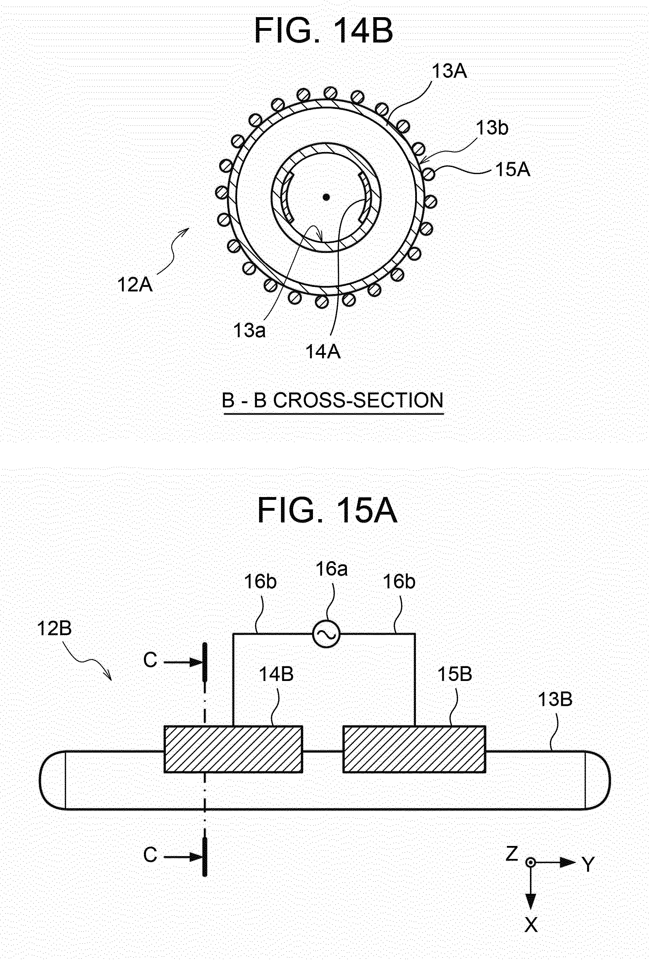

Note that the configuration of the excimer lamp is not limited to the configuration illustrated in FIGS. 13A and 13B. For example, the excimer lamp 12A shown in FIGS. 14A and 14B may be configured to include a double-tube discharge vessel 13A.

The discharge vessel 13A included in the excimer lamp 12A has a cylindrical outer tube and a cylindrical inner tube arranged coaxially with the outer tube inside the outer tube and having an inner diameter smaller than that of the outer tube. The outer tube and the inner tube are sealed at the end portions in the left-right direction in FIG. 14A, and an annular internal space is formed between the outer tube and the inner tube. A discharge gas is enclosed in the internal space.

A membrane first electrode (inner electrode) 14A is provided on the inner wall surface 13a of the inner tube, and a reticulated of mesh second electrode (outer electrode) 15A is provided on the outer wall surface 13b of the outer tube.

The inner electrode 14A and the outer electrode 15A are electrically connected to the high frequency power supply 16a via the power supply line 16b, respectively.

When a high frequency AC voltage is applied between the inner electrode 14A and the outer electrode 15A by the high frequency power supply 16a, a voltage is applied to the discharge gas through the tube of the outer tube and the inner tube, and a dielectric barrier discharge is generated in the discharge space in which the discharge gas is sealed. As a result, the atoms of the discharge gas are excited to generate excited dimers, and excimer emission is generated when the atoms transition to the ground state.

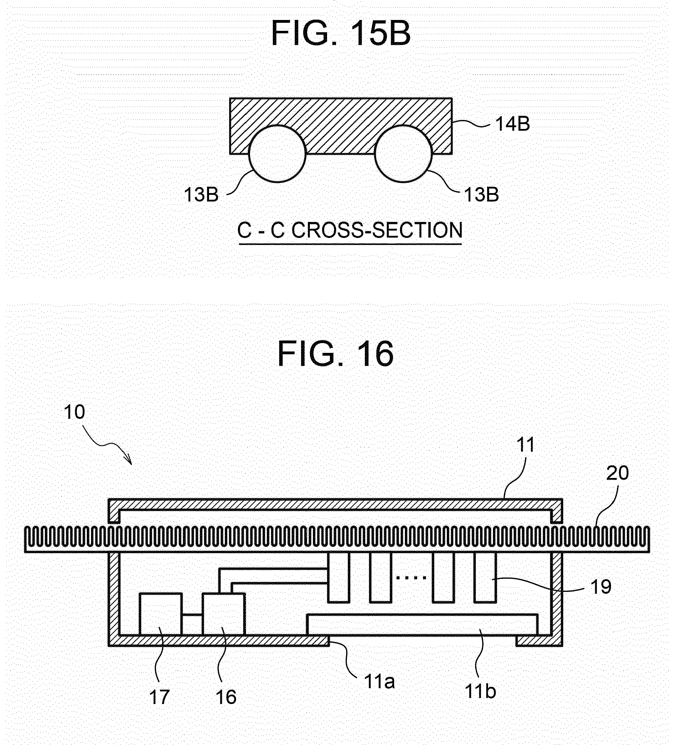

Further, the configuration of the excimer lamp, for example, like the excimer lamp 12B shown in FIGS. 15A and 15B, a pair of electrodes on one side surface of the discharge vessel 13B (first electrode 14B, second electrode 15B) may be arranged. For example, it is assumed that two discharge vessels 13B are arranged side by side in Z-direction in FIG. 15A.

As shown in FIG. 15A, the first electrode 14B and the second electrode 15B are arranged on the side surface of the discharge vessel 13B opposite to the light-extraction surface (the surface in minus X-direction) and spaced apart from each other in the tube axial direction (Y-direction) of the discharge vessel 13B.

The discharge vessel 13B is arranged so as to straddle the two 14B, 15B. Specifically, the two electrodes 14B, 15B are formed with recessed grooves extending in Y-direction, and the discharge vessel 13B is fitted into the recessed grooves of the electrode 14B, 15B.

The first electrode 14B and the second electrode 15B are electrically connected to the high frequency power supply 16a via a power supply line 16b, respectively. When a high frequency AC voltage is applied between the first electrode 14B and the second electrode 15B, an excited dimer is generated in the inner space of the discharge vessel 13B, and the excimer light is emitted from the light extraction surface (plus X-direction surface) of the excimer lamp 12B.

The electrode 14B, 15B may be formed of a metal-material that is reflective to the light emitted from the excimer lamp 12B. In this case, the light emitted from the discharge vessel 13B in minus X-direction can be reflected and advanced in plus X-direction. The electrode 14B, 15B may be made of, for example, aluminum (Al) or stainless-steel.

Since the excimer lamp performs high-frequency lighting by applying high-frequency power as described above, high-frequency noise is generated. However, as described above, the housing 11 that contains the excimer lamp is composed of conductive metal, which can suppress the high-frequency noise from the excimer lamp from being transmitted to the outside of the housing 11. Thus, it is possible to suppress the control command to the control system installed in the vicinity of the ultraviolet irradiation unit 10 from being subjected to disturbance due to the high-frequency noise, and thus it is possible to prevent a defect in the control command.

As described above, in the ultraviolet irradiation device 100 according to the present embodiment, it is preferable to use a KrCI excimer lamp that emits ultraviolet light with a peak wavelength of 222 nm or a KrBr excimer lamp that emits ultraviolet light having a peak wavelength of 207 nm, as an excimer lamp that is an ultraviolet light source.

Both of ultraviolet light with a wavelength of 222 nm emitted from a KrCI excimer lamp and ultraviolet light with a wavelength of 207 nm emitted from a KrBr excimer lamp are light that are safe for humans and animals and that can kill microorganisms and inactivate viruses. Therefore, even if persons or animals exist in the sterilization/inactivation region in the space, the sterilization/inactivation work by the ultraviolet irradiation can be performed.

In the above embodiment, the excimer lamp is used as the ultraviolet light source, but an LED may be used as the ultraviolet light source.

FIG. 16 shows an exemplary ultraviolet irradiation unit 10 using an LED19 as an ultraviolet light source. In FIG. 16, the ultraviolet irradiation unit 10 includes a plurality of LEDs 19.

As described above, the wavelength range of ultraviolet light used for decontamination (sterilization) applications is 200 to 320 nm, and a particularly effective wavelength is around 260 nm in which nucleic acid (DNA, RNA) is highly absorbed.

Therefore, as for the LEDs 19 as an ultraviolet light source mounted on the ultraviolet irradiation unit 10, the LEDs that emit ultraviolet light with a wavelength 200 to 320 nm is also employed. Specifically, for example, aluminum-gallium nitride (AlGaN)-based LEDs, aluminum-nitride (AIN)-based LEDs, or the like may be employed. AlGaN-based LEDs emit light in a deep ultraviolet region (deepUV: DUV) in a wavelength of 200 to 350 nm by changing the composition of the aluminum (Al). In addition, AIN-based LEDs emit ultraviolet light with a peak wavelength of 210 nm.

As AlGaN-based LEDs, it is preferable to adjust the composition of Al so that the center wavelength is within 200 to 237 nm. As described above, ultraviolet light in this wavelength range is safe for humans and animals, and it is possible to appropriately kill microorganisms and inactivate viruses. For example, AlGaN-based LEDs with a central wavelength of 222 nm of emitted ultraviolet light can be obtained by adjusting the composition of Al.

In addition, magnesium-zinc oxide (MgZnO)-based LEDs may be employed as the LED. MgZnO-based LEDs emit light in the deep ultraviolet region (deepUV: DUV) of 190 to 380 nm wavelength-range by changing the composition of the magnesium (Mg).

As MgZnO-based LED, it is preferable to adjust the composition of Mg so that a center wavelength is within 200 to 237 nm.

As described above, ultraviolet light in this wavelength range are safe for humans and animals, and it is possible to appropriately kill microorganisms and inactivate viruses. For example, MgZnO-based LEDs with a central wavelength of 222 nm of emitted ultraviolet light can be obtained by adjusting the composition of Mg.

LEDs that emit the ultraviolet light (particularly, ultraviolet light in the deep ultraviolet region) as described above have a low luminous efficacy of several percent or less and a high heat generation. Further, when the heat generation of LEDs increases, the intensity of the light emitted from LEDs decreases, and the wavelength-shift of the emitted light also occurs. Therefore, in order to suppress the heat increase of LEDs, as shown in FIG. 16, it is preferable to install the LEDs 19 in a cooling member (for example, a heat dissipating fin that dissipates heat) 20.

At this time, as shown in FIG. 16, a part of the cooling member 20 may protrude from the housing 11 of the ultraviolet irradiation unit 10. In this case, the outside air comes into contact with a part of the cooling member 20, and the heat dissipation of the cooling member 20 proceeds efficiently, so that the heat increase of the LEDs 19 can be appropriately suppressed.

It should be noted that AlGaN-based and MgZnO-based LEDs emitting ultraviolet light with a center wavelength of 222 nm emit ultraviolet light in a wavelength range having a certain extent from the center wavelength range, and the light emitted from LEDs includes ultraviolet light with a wavelength that is slightly unsafe to persons and animals. Therefore, as in the case where the ultraviolet light source is an excimer lamp, it is preferable to use a dielectric multilayer film filter (optical filter) that cuts light in a UV-C wavelength range having a wavelength other than the wavelength range 200 to 237 nm.

It should be noted that the optical filter may be one that cuts light in a UV-C wavelength range having a wavelength other than the wavelength 200 to 235 nm, more preferably one that cuts light in a UV-C wavelength range having a wavelength other than 200 to 230 nm. This is also the case when the light source is an excimer lamp.

However, in AIN-LED of emitting ultraviolet light with a center wavelength of 210 nm, the optical filters are not required.

Further, even if the ultraviolet light source is an excimer lamp or an LED, the illuminance of the ultraviolet light at a wavelength that is not safe for humans or animals on the irradiated surface may be equal to or less than an allowable value depending on the illuminance of the ultraviolet light source on the light emitting surface, the distance from the ultraviolet light source to the irradiated surface of the ultraviolet light, or the like. Therefore, in such a case, it is not necessary to provide the optical filter.

Further, in the above embodiment, the case is described in which the maximum allowable ultraviolet irradiation amount is determined using the allowable limit value (TLV) specified in ACGIH and JIS Z 8812, and irradiation amount control is performed, but other safety standards may be considered.

For example, the criteria for evaluating photobiological safety is specified in IEC 62471-2. Photobiological risks are classified into four risk groups according to the degree of biological injury. The risk groups are determined by measuring the irradiance of the light source. Therefore, for example, the irradiation amount control may be performed in consideration of the irradiance of the light source.

Further, in the above embodiment, ultraviolet light in a wavelength range of 200 to 240 nm is used as the light in the wavelength range for inactivating microorganisms and/or viruses, but it is also possible to use, for example, visible light (blue light) with a wavelength of 405 nm. In this case, the ultraviolet irradiation device in the above embodiment is a light irradiation device including a light source that emits light with a center wavelength of 405 nm.

### REFERENCE NUMERALS AND SYMBOLS

10: Ultraviolet irradiation unit
11: Housing
12: Excimer lamp
13: Discharge vessel
14: First electrode
15: Second electrode
16: Power supply unit
17: Control unit
18: Supporting part
19: LED
20: Cooling member
100: Ultraviolet irradiation device
110: Ultraviolet irradiator
120: Control unit
200: Facility
300: Person
1000A; 1000B; 1000C: Inactivation system

## Claims

1. An inactivation method for inactivating microorganisms and/or viruses present in a specific space in which a person is present by emitting light in a wavelength range which inactivates microorganisms and/or viruses into the space, the method comprising:
a detection step of detecting a person present in an irradiation space irradiated with the light;
an acquisition step of acquiring an accumulated irradiation amount which is an accumulated value of an irradiation amount of the light irradiated to the detected person in a period from a predetermined reference time point to a current time point;
a determination step of determining an allowable maximum irradiation amount which is a maximum irradiation amount of the light allowed in the irradiation space by comparing an allowable limit value (TLV: Threshold Limit Value) of an irradiation amount of the light in the wavelength range with the accumulated irradiation amount; and
an irradiation step of controlling a light source for emitting the light and irradiating the light into the irradiation space with the allowable maximum irradiation amount as an upper limit.

2. The inactivation method according to claim 1, wherein the light is ultraviolet light in a wavelength range of 200 to 240 nm.

3. The inactivation method according to claim 1 or 2, wherein in a case where a plurality of persons are detected in the detection step,
in the acquisition step, the accumulated irradiation amount of each of the plurality of persons present in the irradiation space is acquired, and
in the determination step, an amount obtained by subtracting the largest accumulated irradiation amount from the accumulated irradiation amounts of the plurality of persons from the allowable limit value is determined as the allowable maximum irradiation amount.

4. The inactivation method according to any one of claims 1 to 3, wherein the acquisition of the accumulated irradiation amount in the acquisition step and the determination of the allowable maximum irradiation amount in the determination step are performed at a timing at which entry and exit of a person into and from the irradiation space are detected.

5. The inactivation method according to any one of claims 1 to 4, wherein in the irradiation step, an illuminance of the light is controlled so that an irradiation amount of the light does not exceed the allowable maximum irradiation amount.

6. The inactivation method according to any one of claims 1 to 4, wherein in the irradiation step, an irradiation time of the light is controlled so that an irradiation amount of the light does not exceed the allowable maximum irradiation amount.

7. An inactivation system for inactivating microorganisms and/or viruses present in a specific space in which a person is present by emitting light into the space, comprising at least one light irradiation device, the device comprising:
a light irradiation unit comprising a light source emitting light in a wavelength range which inactivates microorganisms and/or viruses; and
a control unit configured to control an irradiation of the light by the light source, wherein
the control unit includes:
a detection unit configured to detect a person present in the irradiation space irradiated with the light from the light source;
an acquisition unit configured to acquire an accumulated irradiation amount which is an accumulated value of an irradiation amount of the light irradiated to a person detected by the detection unit in a period from a predetermined reference time point to a current time point; and
a determination unit configured to determine an allowable maximum irradiation amount which is a maximum irradiation amount of the light allowed in the irradiation space by comparing an allowable limit value (TLV: Threshold Limit Value) of an irradiation amount of the light in the wavelength range with the accumulated irradiation amount acquired by the acquisition unit, and wherein
the control unit controls the light source to irradiate the light into the irradiation space with the allowable maximum irradiation amount determined by the determination unit as an upper limit.

8. The inactivation system according to claim 7, wherein the light is ultraviolet light in a wavelength range of 200 to 240 nm.

9. The inactivation system according to claim 7 or 8, wherein in a case where a plurality of persons are detected by the detection unit,
the acquisition unit acquires the accumulated irradiation amount of each of the plurality of persons present in the irradiation space, and
the determination unit determines an amount obtained by subtracting the largest accumulated irradiation amount from the accumulated irradiation amounts of the plurality of persons acquired by the acquisition unit from the allowable limit value, as the allowable maximum irradiation amount.

10. The inactivation system according to any one of claims 7 to 9, wherein the acquisition of the accumulated irradiation amount by the acquisition unit and the determination of the allowable maximum irradiation amount by the determination unit are performed at a timing at which the detection unit detects entry and exit of a person into and from the irradiation space.

11. The inactivation system according to any one of claims 7 to 10, wherein the control unit controls an illuminance of the light so that an irradiation amount of the light does not exceed the allowable maximum irradiation amount.

12. The inactivation system according to any one of claims 7 to 10, wherein the control unit controls an irradiation time of the light so that the irradiation amount of the light does not exceed the allowable maximum irradiation amount.

13. The inactivation system according to any one of claims 7 to 12, further comprising a recording device which is carried by a person moving in the specific space and records the accumulated irradiation amount of the person in a period from the reference time point to a current time point, wherein
the acquisition unit acquires the accumulated irradiation amount from the recording device carried by a person detected by the detection unit.

14. The inactivation system according to claim 13, wherein the recording device includes a light sensor carried by a person moving in the specific space and configured to measure an illuminance of the light irradiated to the person, and
the recording device is a sensor device which calculates an irradiation amount of the light based on the illuminance of the light measured by the light sensor and an irradiation time of the light, and records the accumulated irradiation amount.

15. The inactivation system according to any one of claims 7 to 12, further comprising a recording device configured to record the accumulated irradiation amounts of all persons moving in the specific space in a time period from the reference time point to the current time point in association with personal identification information for identifying an individual person, wherein
the detection unit identifies a person present in the irradiation space to acquire the personal identification information, and
the acquisition unit acquires, from the recording device, the accumulated irradiation amount associated with the personal identification information of a person identified by the detection unit.

16. The inactivation system according to claim 15, further comprising a light sensor carried by a person moving in the specific space and configured to measure an illuminance of the light irradiated to the person, wherein
the control unit includes a transmission unit configured to acquire an illuminance of the light from the light sensor carried by a person identified by the detection unit, calculate an irradiation amount of the light based on the acquired illuminance of the light and an irradiation time of the light, and transmit the irradiation amount to the recording device in association with the personal identification information of a person identified by the detection unit, and
the recording device records the irradiation amount of the light transmitted by the transmission unit by accumulating the accumulated irradiation amount recorded in association with the personal identification information.

17. The inactivation system according to claim 15, further comprising:
a distance acquisition unit configured to acquire a distance from the light source to an irradiation target of the light, and
a light intensity acquisition unit configured to acquire a light intensity of the light emitted from the light source, wherein
the control unit includes a transmission unit configured to calculate an illuminance of the light irradiated to a person identified by the detection unit based on a distance from the light source to a person identified by the detection unit and a light intensity acquired by the light intensity acquisition unit, calculate an irradiation amount of the light based on the calculated luminance of the light and an irradiation time of the light, and transmit the irradiation amount to the recording device in association with the personal identification information of a person identified by the detection unit, and
the recording device records an irradiation amount of the light transmitted by the transmission unit by accumulating the accumulated irradiation amount recorded in association with the personal identification information.

18. The inactivation system according to claim 17, wherein the distance acquisition unit acquires, as a distance from the light source to the irradiation target, a value obtained by subtracting a reference height, which is a height from a floor to the irradiation target, from a height from the floor to the light source.

19. The inactivation system according to any one of claims 15 to 18, comprising a plurality of the light irradiation devices in the specific space, wherein the accumulated irradiation amount recorded in the recording device is used as shared data of the control unit included in each of the plurality of light irradiation devices.

20. The inactivation system according to any one of claims 13 to 19, wherein the recording device resets the recorded accumulated irradiation amount every predetermined period from the reference time point.

21. The inactivation system according to any one of claims 7 to 20, further comprising a display unit configured to display, for a person moving in the specific space, a time until the accumulated irradiation amount of the person reaches the allowable limit value.

22. The inactivation system according to any one of claims 7 to 21, wherein the light irradiation unit includes a housing having a light emitting window for emitting at least a part of the light emitted from the light source, and
the light emitting window is equipped with an optical filter which blocks transmission of UV-C wave on a longer side of a wavelength than 237 nm.

23. The inactivation system according to any one of claims 7 to 22, wherein the light source is one of an excimer lamp and an LED.

24. The inactivation system according to any one of claims 7 to 23, wherein the light source emits ultraviolet light with a center wavelength of 222 nm.

25. The inactivation system according to any one of claims 7 to 24, wherein the light source is an LED, and the LED is one of an aluminum gallium nitride (AlGaN)-based LED, an aluminum nitride (AIN)-based LED, and a magnesium zinc oxide (MgZnO)-based LED.

26. The inactivation system according to any one of claims 7 to 25, wherein the light source is an LED, and the light irradiation unit includes a cooling member for cooling the LED.

27. The inactivation system according to any one of claims 7 to 24, wherein the light source is an excimer lamp, and the light irradiation unit includes a housing which contains the excimer lamp and is made of a conductive metal.
